# EUROPEAN PATENT APPLICATION

(11) **EP 4 234 572 A1**
(43) Date of publication of application: **30.08.2023**
(21) Application number: 21882001.7
(22) Date of filing: 19.10.2021
(51) Int. Cl.: C07K 14/195, A61K 39/09, A61K 39/385, A61P 31/04, A61P 11/00

(54) **PROTEOGLYCAN CONJUGATE AND APPLICATION THEREOF**

(30) Priority: 20.10.2020 CN 202011126136
(71) Applicant: Shanghai Reinovax Biologics Co., Ltd, Shanghai 201203 (CN); Shanghai Microdom Biotech Co., Ltd, Shanghai 201413 (CN)
(72) Inventor: ZHU, Xianchao, Shanghai 201203 (CN); CHEN, Huagen, Shanghai 201203 (CN); XIONG, Xishuang, Shanghai 201203 (CN); LI, Ying, Shanghai 201203 (CN); WANG, Juanjuan, Shanghai 201203 (CN); LIU, Chang, Shanghai 201203 (CN); XIA, Qingfeng, Shanghai 201203 (CN); MAO, Yizhi, Shanghai 201203 (CN); WANG, Zhu, Shanghai 201203 (CN); SHEN, Enhua, Shanghai 201203 (CN)
(74) Representative: Nederlandsch Octrooibureau
(86) International application number: PCT/CN2021/124705
(87) International publication number: WO 2022/083585

(57) **Abstract**

Provided is a tetanus toxin protein variant, the tetanus toxin protein variant comprising a C fragment of the tetanus toxin protein and a partial fragment of a translocation region of the tetanus toxin protein. Further provided is a proteoglycan conjugate, the proteoglycan conjugate comprising a polysaccharide from Streptococcus pneumoniae and a truncated tetanus toxin protein. Also provided is a method for improving immunogenicity, the method comprising the following steps: providing a proteoglycan conjugate comprising a polysaccharide from Streptococcus pneumoniae and a truncated tetanus toxin protein.

## Description

### Technical Field

The present application relates to the field of biomedicine, in particular to a polysaccharide protein conjugate and use thereof. The polysaccharide protein conjugate comprises a polysaccharide of Streptococcus pneumoniae and a truncated tetanus toxin protein. The polysaccharide protein conjugate can improve the immunogenicity of Streptococcus pneumoniae capsular polysaccharide.

### Background Technology

Streptococcus pneumonia (pneumococcus), formerly known as "Diplococcus pneumoniae", is a Gram-positive diplococcus which has more than 91 serotypes according to the composition of the capsular polysaccharide, wherein the capsular polysaccharide is an important pathogenic factor. Pneumococcal infection is one of the serious public health problems worldwide. According to the World Health Organization, in 2005, 1.6 million people died of pneumococcus-related disease every year in the world, including 700,000 to 1,000,000 children under the age of 5, most of them lived in developing countries. It is clear that the pneumococcal infection has been seriously endangering the health of children. In developed countries, pneumococcal infection mainly occurs in children under 2 years of age and the elderly, as well as immunocompromised individuals of all age groups.

The pneumococcal polysaccharide conjugate vaccine is produced by conjugating pneumococcal polysaccharides with carrier proteins, and the carrier proteins play a very important role in improving the immunogenicity of pneumococcal polysaccharides. In numbers of clinical studies using Streptococcus pneumoniae vaccines domestically and internationally, the termination of the clinical study due to low immunogenicity or lack of effective protection is not uncommon (Marilla G Lucero et al, The Pediatric Infectious Disease Journal. 28 (6) : 455 462 ,JUN 2009) (Jan Poolman et al, Vaccine. 2009 May 21;27(24):3213-22). Therefore, there is an urgent need to develop strategies and vaccines that can induce better immunogenicity and protection against Streptococcal infection.

### Summary of the Invention

The present application provides a tetanus toxin protein variant. The tetanus toxin protein variant comprises the segment C of the tetanus toxin protein and a partial segment of the translocation region of the tetanus toxin protein, can be used as a carrier protein to conjugate with the polysaccharide of the Streptococcus pneumoniae, and has the effect of improving the immunogenicity of the polysaccharide of Streptococcus pneumoniae. The present application also provides a polysaccharide protein conjugate comprising the polysaccharide from Streptococcus pneumoniae and a truncated tetanus toxin protein, and the polysaccharide conjugate has significantly better immunogenicity. The present application also provides a method for improving immunogenicity, and the method comprises the following steps: providing a polysaccharide protein conjugate comprising the polysaccharide from Streptococcus pneumoniae and the truncated tetanus toxin protein, and the method can improve the immunogenicity for the protection against Streptococcus pneumoniae.

In one aspect, the present application provides a tetanus toxin protein variant, which comprises the segment C of the tetanus toxin protein and the partial segment of the translocation region of the tetanus toxin protein.

In certain embodiments, the partial segment of the translocation region comprises the T cell epitope P2 of the translocation region of the tetanus toxin protein.

In certain embodiments, the partial segment of the translocation region comprises amino acids 829-864 of the tetanus toxin protein.

In certain embodiments, the partial segment of the translocation region comprises the amino acid sequence shown in any one of SEQ ID NO: 7-8.

In certain embodiments, the segment C of the tetanus toxin protein comprises the amino acid sequence shown in SEQ ID NO: 3.

In certain embodiments, the tetanus toxin protein variant comprises the amino acid sequence shown in any one of SEQ ID NO: 1-2.

In another aspect, the present application provides a polysaccharide protein conjugate comprising the polysaccharide from Streptococcus pneumoniae and a truncated tetanus toxin protein (TTD).

In certain embodiments, the polysaccharide is derived from Streptococcus pneumoniae capsular polysaccharides.

In certain embodiments, the polysaccharide has more than one serotype of Streptococcus pneumoniae.

In certain embodiments, the polysaccharide is selected from any serotype of the group of Streptococcus pneumoniae serotypes consisting of: 1, 2, 3, 4, 5, 6A, 6B, 7F, 8, 9N, 9V, 10A, 11A, 12F, 14, 15B, 17F, 18C, 19A, 19F, 20, 22F, 23F, and 33F.

In certain embodiments, the truncated tetanus toxin protein comprises the segment C of the tetanus toxin protein.

In certain embodiments, the truncated tetanus toxin protein comprises the segment C of the tetanus toxin protein, wherein the polysaccharide is selected from any serotype of the group of Streptococcus pneumoniae serotypes consisting of: 1, 3, 5, 6A, 6B, 7F, 10A, 12F, 15B, 19A, 19F, and 33F.

In certain embodiments, the segment C of the tetanus toxin protein comprises the amino acid sequence shown in SEQ ID NO: 3.

In certain embodiments, the truncated tetanus toxin protein comprises the segment C of the tetanus toxin protein and the partial segment of the translocation region of the tetanus toxin protein.

In certain embodiments, the partial segment of the translocation region comprises the universal T cell epitope P2 of the translocation region of the tetanus toxin protein.

In certain embodiments, the partial segment of the translocation region comprises amino acids 829-864 of the tetanus toxin protein.

In certain embodiments, the partial segment of the translocation region comprises the amino acid sequence shown in any one of SEQ ID NO: 7-8.

In certain embodiments, the truncated tetanus toxin protein comprises the amino acid sequence shown in any one of SEQ ID NO: 1-3.

In certain embodiments, the mass ratio of the polysaccharide to the truncated tetanus toxin protein is 0.4-2.5.

In certain embodiments, the mass ratio of the Streptococcus pneumoniae serotype 1 polysaccharide to the truncated protein is 0.4-2.5.

In certain embodiments, the mass ratio of the Streptococcus pneumoniae serotype 3 polysaccharide to the truncated protein is 0.4-2.5.

In certain embodiments, the mass ratio of the Streptococcus pneumoniae serotype 5 polysaccharide to the truncated protein is 0.4-2.5.

In certain embodiments, the mass ratio of the Streptococcus serotype 6A pneumoniae polysaccharide to the truncated protein is 0.4-2.5.

In certain embodiments, the mass ratio of the Streptococcus serotype 6B pneumoniae polysaccharide to the truncated protein is 0.4-2.5.

In certain embodiments, the mass ratio of the Streptococcus serotype 7F pneumoniae polysaccharide to the truncated protein is 0.4-2.5.

In certain embodiments, the mass ratio of the Streptococcus serotype 10A pneumoniae polysaccharide to the truncated protein is 0.4-2.5.

In certain embodiments, the mass ratio of the Streptococcus serotype 12F pneumoniae polysaccharide to the truncated protein is 0.4-2.5.

In certain embodiments, the mass ratio of the Streptococcus serotype 15B pneumoniae polysaccharide to the truncated protein is 0.4-2.5.

In certain embodiments, the mass ratio of the Streptococcus serotype 19A pneumoniae polysaccharide to the truncated protein is 0.4-2.5.

In certain embodiments, the mass ratio of the Streptococcus serotype 19F pneumoniae polysaccharide to the truncated protein is 0.4-2.5.

In certain embodiments, the mass ratio of the Streptococcus serotype 33F pneumoniae polysaccharide to the truncated protein is 0.4-2.5.

In certain embodiments, for the polysaccharide protein conjugate, the polysaccharide and the protein variant can be conjugated by a conjugation method

In certain embodiments, the conjugation method comprises any one of the following methods: hydrogen bromide method, CDAP method, reductive amination method.

In another aspect, the present application provides a method for improving immunogenicity, which includes the following steps: providing a polysaccharide protein conjugate comprising the polysaccharide from Streptococcus pneumoniae and a truncated tetanus toxin protein.

In certain embodiments, the polysaccharide is derived from Streptococcus pneumoniae capsular polysaccharides.

In certain embodiments, the polysaccharide has more than one serotype of Streptococcus pneumoniae.

In certain embodiments, the polysaccharide is any serotype of Streptococcus pneumoniae selected from the group consisting of: 1, 2, 3, 4, 5, 6A, 6B, 7F, 8, 9N, 9V, 10A, 11A, 12F, 14, 15B, 17F, 18C, 19A, 19F, 20, 22F, 23F, and 33F.

In certain embodiments, the truncated tetanus toxin protein comprises the segment C of the tetanus toxin protein.

In certain embodiments, the truncated tetanus toxin protein comprises the segment C of the tetanus toxin protein, wherein the polysaccharide is any serotype of Streptococcus pneumoniae selected from the group consisting of: 1, 3, 5, 6A, 6B, 7F, 10A, 12F, 15B, 19A, 19F, and 33F.

In certain embodiments, the segment C of the tetanus toxin protein comprises the amino acid sequence shown in SEQ ID NO: 3.

In certain embodiments, the truncated tetanus toxin protein comprises the segment C of the tetanus toxin protein and the partial segment of the translocation region of the tetanus toxin protein.

In certain embodiments, the partial segment of the translocation region comprises the universal T cell epitope P2 of the translocation region of the tetanus toxin protein.

In certain embodiments, the partial segment of the translocation region of the tetanus toxin protein comprises amino acids 829-864 of the tetanus toxin protein.

In certain embodiments, the truncated tetanus toxin protein comprises the amino acid sequence shown in any one of SEQ ID NO: 1-3.

In certain embodiments, the mass ratio of the Streptococcus pneumoniae polysaccharide to the truncated tetanus toxin protein is 0.4-2.5.

In certain embodiments, the mass ratio of the Streptococcus pneumoniae serotype 1 polysaccharide to the truncated protein is 0.4-2.5.

In certain embodiments, the mass ratio of the Streptococcus pneumoniae serotype 3 polysaccharide to the truncated protein is 0.4-2.5.

In certain embodiments, the mass ratio of the Streptococcus pneumoniae serotype 5 polysaccharide to the truncated protein is 0.4-2.5.

In certain embodiments, the mass ratio of the Streptococcus pneumoniae serotype 6A polysaccharide to the truncated protein is 0.4-2.5.

In certain embodiments, the mass ratio of the Streptococcus pneumoniae serotype 6B polysaccharide to the truncated protein is 0.4-2.5.

In certain embodiments, the mass ratio of the Streptococcus pneumoniae serotype 7F polysaccharide to the truncated protein is 0.4-2.5.

In certain embodiments, the mass ratio of the Streptococcus pneumoniae serotype 10A polysaccharide to the truncated protein is 0.4-2.5.

In certain embodiments, the mass ratio of the Streptococcus pneumoniae serotype 12F polysaccharide to the truncated protein is 0.4-2.5.

In certain embodiments, the mass ratio of the Streptococcus pneumoniae serotype 15B polysaccharide to the truncated protein is 0.4-2.5.

In certain embodiments, the mass ratio of the Streptococcus pneumoniae serotype 19A polysaccharide to the truncated protein is 0.4-2.5.

In certain embodiments, the mass ratio of the Streptococcus pneumoniae serotype 19F polysaccharide to the truncated protein is 0.4-2.5.

In certain embodiments, the mass ratio of the Streptococcus pneumoniae serotype 33F polysaccharide to the truncated protein is 0.4-2.5.

In certain embodiments, the method comprises conjugating the polysaccharide and the protein variant by a conjugation method.

In certain embodiments, the conjugation method comprises any one of the following methods: hydrogen bromide method, CDAP method, reductive amination method.

In certain embodiments, the improving the immunogenicity of the bacterial polysaccharide comprises that the polysaccharide protein conjugate has a higher immunogenicity than the polysaccharide CRM197 conjugate.

In certain embodiments, the higher immunogenicity is detected by an animal immunization experiment.

In certain embodiments, the animal immunization experiment comprises the following steps: preparing the polysaccharide protein conjugate and adjuvant into an immune antigen.

In certain embodiments, the injection method of the immune antigen comprises intraperitoneal injection, subcutaneous injection, intramuscular injection and/or intravenous injection.

In certain embodiments, the animal immunization experiment comprises the following steps: detecting the antibodies in the sera of the obtained immunized animals by ELISA.

In certain embodiments, the animal immunization experiment comprises the following steps: performing the opsonophagocytic killing assay on the serum of the obtained immunized animals.

In certain embodiments, the animals comprise mice, rats and/or rabbits.

In certain embodiments, the adjuvant comprises aluminum hydroxide, aluminum phosphate and/or Freund's adjuvant and the like.

In another aspect, the present application provides a nucleic acid molecule which comprises a sequence encoding the tetanus toxin protein variant.

In certain embodiments, the nucleic acid molecule comprises the nucleotide sequences shown in SEQ ID NO: 4-6.

In another aspect, the present application provides a vector which comprises the nucleic acid molecule.

In another aspect, the present application provides a cell which comprises the nucleic acid molecule or the vector.

In another aspect, the present application provides a carrier protein which comprises the tetanus toxin protein variant

In another aspect, the present application provides a pharmaceutical composition, which comprises the polysaccharide protein conjugate and optionally a pharmaceutically acceptable adjuvant.

In another aspect, the present application provides use of the tetanus toxin protein variant for the preparation of medicaments.

In another aspect, the present application also provides use of the polysaccharide conjugate for the preparation of medicaments.

In certain embodiments, the medicament is used for preventing and/or treating Streptococcus pneumoniae disease.

In certain embodiments, the Streptococcus pneumoniae disease comprises pneumonia, sepsis, meningitis and/or otitis media.

In another aspect, the present application provides a vaccine which comprises the polysaccharide protein conjugate, the pharmaceutical composition and optionally a pharmaceutically acceptable adjuvant.

In another aspect, the present application provides a method for preventing Streptococcus pneumoniae disease which comprises administering the polysaccharide protein conjugate, the nucleic acid molecule, the vector, the cell, the carrier protein, the pharmaceutical composition and/or the vaccine to a subject in need.

In certain embodiments, the Streptococcus pneumoniae disease comprises pneumonia, sepsis, meningitis and/or otitis media.

In another aspect, the present application provides use of the polysaccharide conjugate as an antigen for the preparation of antibodies.

In certain embodiments, the antibody is used for diagnostic typing of an isolated bacterial strain.

In another aspect, the present application provides a kit which comprises the polysaccharide conjugate.

In certain embodiments, the kit is used for diagnostic typing of an isolated bacterial strain.

### Brief Description of the Figures

The particular features of the invention to which this application relates are set forth in the appended claims. The features and advantages of the present invention to which this application relates can be better understood with reference to the exemplary embodiments described in details hereinafter and the accompanying drawings. A brief description of the accompanying drawings is as follows:
Figure 1 shows the electrophoresis of the expression plasmid of the truncated tetanus toxin proteins (TTD) described herein.
Figure 2 shows the electrophoresis of the TTD proteins described herein after being expressed in *Escherichia coli.*
Figure 3 shows the electrophoresis of the purified TTD proteins described herein.
Figure 4 shows the SEC-HPLC profile of the purified TTD protein described herein.
Figure 5 shows the antibody titers in the mouse serum after immunization with the serotype-3-polysaccharide conjugates with TTD-1, 2, 3 described herein and the serotype-3-polysaccharide conjugate with CRM197.
Figure 6 shows the opsonophagocytosis function of specific antibodies in the mouse serum after immunization with the serotype-3-polysaccharide-TTD-1 conjugate described herein and the serotype-3-polysaccharide-CRM197 conjugate.
Figure 7 shows the antibody titers in the mouse serum after immunization with the serotype-6B-polysaccharide-TTD-1 conjugate described herein and the serotype-6B-polysaccharide-CRM197 conjugate.
Figure 8 shows the opsonophagocytosis function of specific antibodies in the mouse serum after immunization with the serotype-6B-polysaccharide-TTD-1 conjugate described herein and the serotype-6B-polysaccharide-CRM197 conjugate.
Figure 9 shows the antibody titers in the mouse serum after immunization with the serotype-15B-polysaccharide-TTD-3 conjugate described herein and the serotype-15B-polysaccharide-CRM197 conjugate.
Figure 10 shows the opsonophagocytosis function of specific antibodies in the mouse serum after immunization with the serotype-15B-polysaccharide-TTD-3 conjugate described herein and the serotype-15B-polysaccharide-CRM197 conjugate.
Figure 11 shows the antibody titers in the mouse serum after immunization with the serotype-6A-polysaccharide-TTD-3 conjugate described herein and the serotype-6A-polysaccharide-CRM197 conjugate.
Figure 12 shows the opsonophagocytosis function of specific antibodies in the mouse serum after immunization with the serotype-6A-polysaccharide-TTD-3 conjugate described herein and the serotype-6A-polysaccharide-CRM197 conjugate.
Figure 13 shows the antibody titers in the rabbit serum after immunization with the serotype-7F-polysaccharide-TTD-1 conjugate described herein and the serotype-7F-polysaccharide-CRM197 conjugate.
Figure 14 shows the opsonophagocytosis function of specific antibodies in the mouse serum after immunization with the serotype-7F-polysaccharide-TTD-3 conjugate described herein and the serotype-7F-polysaccharide-CRM197 conjugate .
Figure 15 shows the antibody titers in the rabbit serum after immunization with the serotype-10A-polysaccharide-TTD-2 conjugate described herein and the serotype-10A-polysaccharide-CRM197 conjugate.
Figure 16 shows the opsonophagocytosis function of specific antibodies in the mouse serum after immunization with the serotype-10A-polysaccharide-TTD-2 conjugate described herein and the serotype-10A-polysaccharide-CRM197 conjugate.
Figure 17 shows the antibody titers in the rabbit serum after immunization with the serotype-19A-polysaccharide-TTD-1 conjugate described herein and the serotype-19A-polysaccharide-CRM197 conjugate.
Figure 18 shows the opsonophagocytosis function of specific antibodies in the rabbit serum after immunization with the serotype-19A-polysaccharide-TTD-1 conjugate described herein and the serotype-19A-polysaccharide-CRM 197 conjugate.
Figure 19 shows the antibody titers in the rabbit serum after immunization with the serotype-1-polysaccharide-TTD-3 conjugate described herein and the serotype-1-polysaccharide-CRM197 conjugate.
Figure 20 shows the opsonophagocytosis function of specific antibodies in the rabbit serum after immunization with the serotype-1-polysaccharide-TTD-3 conjugate described herein and the serotype-1-polysaccharide-CRM197 conjugate.
Figure 21 shows the antibody titers in mouse serum after immunization with the serotype-5, 12F, 33F, 19F-polysaccharide-TTD conjugates described herein and corresponding polysaccharide-CRM197 conjugates.

### Detailed Description

The implementation of the invention of the present application will be described in the following specific examples, and those skilled in the art can easily understand other advantages and effects of the invention of the present application from the content disclosed in this specification.

### Definition of Terms

In this application, the term "tetanus", also known as "clostridium tetani", generally refers to bacteria capable of causing infectious and serious diseases. It can affect the nervous system of an infected person by synthesizing a toxin protein. The toxin protein produced by tetanus is called "tetanus toxin protein", "tetani toxoid" or "tetanospasm toxin". The tetanus toxin protein may comprise three domains (segments) A, B and C, and the molecular weight of each segment may be about 50 kDa. For example, the segment A can be a catalytic domain having an endopeptidase activity. For example, the segment B can be a translocation domain. For example, the segment C may be a domain capable of binding to a receptor (Ana C. Calvo, Int. J. Mol. Sci. 2012, 13, 6883-6901; doi:10.3390/ijmsl3066883). For example, the segment B may comprise the amino acid sequence shown in SEQ ID NO:7. For example, the segment B may comprise the amino acid sequence shown in SEQ ID NO:8. For example, the segment C may comprise the amino acid sequence shown in SEQ ID NO:3. In the present application, the term "the translocation region of the tetanus toxin protein" may refer to the segment B of the tetanus toxin. In the present application, the term "the segment C of the tetanus toxin protein" may refer to the segment C of the tetanus toxin protein.

In this application, the term "protein variant" and "variant" generally refer to a compound having sequence homology to a natural biologically active protein or polypeptide. The protein variant described herein may include proteins having altered amino acid sequences by addition (including insertions), deletions, modifications and/or substitutions of one or more amino acid residues while retaining at least one biological active. For example, a variant may have at least about 0%, at least about 65%, at least about 70%, at least about 75%, at least about 80%, at least about 85%, at least about 90%, at least about 95%, at least about 96%, at least about 97%, at least about 98%, or at least about 99% sequence identity. Variants can be naturally occurring or non-naturally occurring. Non-naturally occurring variants can be generated using techniques known in the art. Protein variants may contain conservative or non-conservative amino acid substitutions, deletions or additions. In the present application, the "tetanus toxin protein variant" may refer to a protein in which part of the amino acid sequence of the tetanus toxin protein is deleted.

In this application, the term "truncated" generally refers to anything that is less than the whole. In the present application, the "truncated tetanus toxin protein (TTD)" may refer to a compound whose the amino acid sequence is less than the entire sequence of the tetanus toxin. For example, the truncated protein is at least about 60%, at least about 65%, at least about 70%, at least about 75%, at least about 80%, at least about 85%, at least about 90%, at least about 95%, at least About 96%, at least about 97%, at least about 98%, or at least about 99% less than the amino acid sequence of the parent toxin. Truncated proteins can be naturally occurring or non-naturally occurring. Non-naturally occurring truncated proteins can be generated using techniques known in the art.

In this application, the term "T cell epitope" generally refers to a polypeptide or protein segment that can be recognized by T lymphocytes. The "T cell epitope P2" generally refers to a polypeptide of the tetanus toxin protein that can be recognized by T cells, which may include p2, p4 and/or p30. For example, p2 may have a DR molecule that recognizes all MHC (histocompatibility complex molecules). The T cell epitope p2 may comprise the 830-844 amino acid sequence of the tetanus toxin protein. For example, p30 can be combined with a large number of different MHC II, which can be recognized by T cells and has immunogenic properties. The T cell epitope p30 may comprise the amino acid sequence at positions 947-967 of the tetanus toxin protein. The T cell epitope p4 may comprise the amino acid sequence at positions 1273-1284 of the tetanus toxin protein. In the present application, for example, the protein variant may comprise the "T cell epitope p2" region.

In this application, the terms "Streptococcus pneumoniae capsular polysaccharide", "Streptococcus pneumoniae polysaccharide" and "pneumococcal polysaccharide" are used interchangeably and generally refer to the loose mucous material on the surface of Streptococcus pneumoniae. Streptococcus pneumoniae polysaccharide induces the body's immune response mainly independent of T cells. In this application, the immune response may include the process of immune cells recognizing, activating, proliferating and differentiating antigen molecules and producing immune substances to produce specific immune effects after the body is stimulated by antigens. The immune response may include a series of physiological reactions such as antigen presentation, lymphocyte activation, immune molecule formation, and immune effect generation. Streptococcus pneumoniae can be divided into different serotypes according to the antigenicity of capsular polysaccharides. In some embodiments, Streptococcus pneumoniae can contain 91 serotypes depending on the capsular polysaccharide antigens. The main pathogenic serotypes of Streptococcus pneumoniae can include the following 24 serotypes, namely: 1, 2, 3, 4, 5, 6A, 6B, 7F, 8, 9N, 9V, 10A, 11A, 12F, 14, 15B, 17F, 18C, 19A, 19F, 20, 22F, 23F and 33F.

In some embodiments, the capsular polysaccharide of Streptococcus pneumoniae may comprise repeating oligosaccharide units which may comprise up to 8 saccharide residues. In some embodiments, the capsular polysaccharide can be a full length polysaccharide. In some embodiments, the capsular polysaccharide may be one oligosaccharide unit or a saccharide chain of repeating oligosaccharide units shorter than the natural length.

In this application, the term "Streptococcus pneumoniae disease" generally refers to diseases caused by Streptococcus pneumoniae infection, including but not limited to childhood pneumonia, meningitis, bacteremia, acute otitis media and sinusitis.

In this application, the term "carrier protein" generally refers to a protein, protein homologue or polypeptide that can combine with saccharides or polysaccharides from microorganisms, carry the saccharides or polysaccharides from microorganisms into the body of a subject and cause an immune response. Conjugation of the saccharide to the carrier protein enhances the immunogenicity of the saccharide because it converts the saccharide from a thymus-independent antigen to a thymus-dependent antigen, thereby triggering immune memory. The thymus-dependent antigen can also be called the T-cell-dependent antigen; the thymus-independent antigen can also be called the T-cell-independent antigen. For children, the elderly, and immunocompromised persons, T cell-dependent antigens can generate an effective immune response and produce antibodies that can better maintain the immune effect. In this application, the term "polysaccharide protein conjugate" refers to a substance with a single structure produced by combining the carrier protein with saccharides or polysaccharides from bacteria.

In this application, the term "conjugate" or "conjugation" refers to the covalent or non-covalent combination of two parts of matter to form a single structure, wherein the first part is an antigen, especially a polysaccharide, and the second part is an immunogen carrier, such as a carrier protein. The association can be achieved through covalent chemical bonds between the molecules or through the use of linking groups, such as adipate dihydrazide.

In this application, the term "immunogenicity" generally refers to the properties capable of eliciting an immune response, including but not limited to the properties capable of stimulating cell activation, proliferation, differentiation, production of immune effector antibodies and sensitization of lymphocytes. In this application, the higher immunogenicity includes but is not limited to higher antibody titer, more serotype-specific antibodies and higher bactericidal efficiency in the serum of the subject after immunization of the subject.

In this application, the term "the mass ratio of a polysaccharide to a protein" can usually be used to measure the degree of glycosylation modification of proteins in conjugates, and different mass ratios affect the immunogenicity of conjugates, and free polysaccharides and free proteins all belong to the substrates of the conjugation reaction, and the free capsular polysaccharide in the conjugate will reduce the immune response of the conjugate, and the excess free protein will also inhibit the immune response. In some embodiments, the molecular size distribution of the conjugate is directly related to the immunogenicity of the product, and it is also an important indicator to measure the conjugation process and the stability of the conjugate.

In this application, the term "vector" generally refers to a vector that contains the regulatory sequences necessary for the transcription and translation of one or more cloned nucleic acid molecules, and thus allows the transcription and cloning of the nucleic acid molecule. The vector may contain one or more regulatory sequences operably linked to the nucleic acid molecule, which regulatory sequences may be selected according to the type of host cell used. Regulatory sequences include promoters, enhancers, and other expression control elements, such as polyadenylation (poly(A)+) sequences. Other vector components may include, but are not limited to, one or more of the following: a signal sequence, an origin of replication, one or more selection genes, and a transcription termination sequence.

In this application, the term "pharmaceutical composition" generally refers to a composition suitable for administration to a subject in need thereof. For example, the pharmaceutical composition described in this application may comprise the polysaccharide protein conjugate described herein and a pharmaceutically acceptable carrier.

As used herein, the term "vaccine" refers to an agent or composition containing active ingredients effective to induce a therapeutic degree of immunity in a subject against a particular pathogen or disease. In the present application, the vaccine may comprise bacterial polysaccharide and carrier protein. In this application, the vaccine may also contain other immunogenic active components. In this application, the combination of bacterial polysaccharide and protein can also be used as an active component in a multi-component vaccine. In this application, the "vaccine" may also comprise a pharmaceutical composition, and thus generally includes a pharmaceutically acceptable diluent, carrier or excipient. It may or may not contain other active ingredients. In some embodiments, it may be a combination vaccine that additionally includes other components that induce an immune response, such as other proteins against bacterial polysaccharides and/or components against other infectious agents.

In this application, the term "ELISA" generally refers to enzyme-linked immunosorbent assay. It can contain various protocols for immunodetection. For example, ELISA methods may include sandwich ELISA, bridging ELISA, ELISA direct method, ELISA indirect method and the like. In some embodiments, ELISA immunoassays can be manual assays or can be performed by automated means. For example, in this application, ELISA method can be used to evaluate the immunogenicity of Streptococcus pneumoniae polysaccharide.

In this application, the term "opsonophagocytic killing assay" is also called "OPA", which generally refers to the assay of promoting phagocytic phagocytosis of bacteria and other particulate antigens by antibodies and complements. The antibodies include antibodies produced from a subject immunized with an antigen. Said antibodies are contained in serum. The phagocytic cells include phagocytic cells or phagocytic cells differentiated from cells with differentiation ability (for example, HL (Hela cells)-60). The bacteria include Streptococcus pneumoniae.

In the present application, the terms "subject" or "individual" or "animal" or "patient" are used interchangeably to refer to a subject, such as a mammalian subject, in need of administration of the pharmaceutical composition of the present application. Animal subjects include humans, non-human primates, dogs, cats, guinea pigs, rabbits, rats, mice, horses, cattle, cows, and the like, e.g., mice, e.g., rats, e.g., rabbits. Wherein the rabbit may be a large rabbit, for example, a New Zealand white rabbit.

In the present application, the term "homology" generally refers to an amino acid sequence or a nucleotide sequence having certain homology with the compared amino acid sequence and the compared nucleotide sequence. The term "homology" may be equated with sequence "identity". The term "homology" may be equated with sequence "identity". Homologous sequences may include amino acid sequences that are at least 80%, 85%, 90%, 99.1%, 99.2%, 99.3%, 99.4%, 99.5%, 99.6%, 99.7%, 99.8%, or 99.9% identical to the subject sequence. Typically, a homologue will comprise the same active site, etc., as the subject amino acid sequence. Homology can be considered in terms of similarity (i.e. amino acid residues with similar chemical properties/functions), or it can be expressed in terms of sequence identity. In the present application, the sequence having a percentage identity to any one of the SEQ ID NO of the mentioned amino acid sequences or nucleotide sequences refers to the sequences having the percent identity over the entire length of the mentioned SEQ ID NO.

To determine sequence identity, sequence alignment can be performed by various means known to those skilled in the art, for example, using BLAST, BLAST-2, ALIGN, NEEDLE or Megalign (DNASTAR) software, among others. Those skilled in the art can determine appropriate parameters for alignment, including any algorithms needed to achieve optimal alignment across the full-length sequences being compared.

In this application, the term "and/or" should be understood as meaning any one of the optional items or both of the optional items.

In this application, the term "comprise" or "include" generally means including specifically specified features, but not excluding other elements.

In this application, the term "about" generally refers to a range of 0.5%-10% above or below the specified value, such as within the range of 0.5%, 1%, 1.5%, 2%, 2.5%,. 3%, 3.5%, 4%, 4.5%, 5%, 5.5%, 6%, 6.5%, 7%, 7.5%, 8%, 8.5%, 9%, 9.5%, or 10% above or below the specified value.

### Summary of the Invention

### Protein variant

In one aspect, the present application provides a tetanus toxin protein variant (Tetanus toxin Domain, TTD), and compared with the amino acid sequence of the wild-type tetanus toxin protein (NBCI accession number WP011100836), the tetanus toxin protein variant comprises the segment C of the tetanus toxin protein and the partial segment of the translocation region of the tetanus toxin protein.

In some embodiments, the tetanus toxin protein variant is obtained by removing the N-terminal amino acid sequence of the tetanus toxin protein variant through genetic engineering recombinant protein technology to obtain a new protein segment that does not contain the N-terminal partial amino acid sequence. The segment A of the N-terminal of the tetanus toxin protein can exert toxic effects by inhibiting the release of neurotransmitters, so in this application, the toxic N-terminal segment is removed by common techniques in the art.

In certain instances, the segment C of the tetanus toxin protein comprises the amino acid sequence shown in SEQ ID NO:3. In some embodiments, the segment C of the tetanus toxin protein may comprise at least about 70% (e.g., at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% %, or at least about 100%) sequence identity to the amino acid sequence set forth in SEQ ID NO:3.

In some embodiments, the partial segment of the translocation region of the tetanus toxin protein comprises the T cell epitope P2 of the translocation region of the tetanus toxin protein.

In some embodiments, the partial segment of the translocation region of the tetanus toxin protein comprises amino acids 829-864 of the tetanus toxin protein.

In some embodiments, the partial segment of the translocation region of the tetanus toxin protein may comprise 1-36 amino acids, for example: 1 amino acid, 2 amino acids, 3 amino acids , 4 amino acids, 5 amino acids, 6 amino acids, 7 amino acids, 8 amino acids, 9 amino acids, 10 amino acids, 11 amino acids, 12 amino acids, 13 amino acids, 14 amino acids, 15 amino acids, 16 amino acids, 17 amino acids, 18 amino acids, 19 amino acids, 20 amino acids, 21 amino acids, 22 amino acids, 23 amino acids, 24 amino acids, 25 amino acids, 26 amino acids, 27 amino acids, 28 amino acids , 29 amino acids, 30 amino acids, 31 amino acids, 32 amino acids, 33 amino acids, 34 amino acids, 35 amino acids or 36 amino acids, of the translocation region of the tetanus toxin protein.

In some embodiments, the amino acids 829-864 are amino acid residues 1-36 of the amino acid sequence shown in SEQ ID NO: 1.

In some embodiments, for example, the partial segment of the translocation region of the tetanus toxin protein comprises amino acids 830-864, amino acids 831-864, amino acids 832-864, amino acids 833-864, amino acids 834-864, amino acids 835-864, amino acids 836-864, amino acids 837-864, 838-864 amino acids, 839-864 amino acids, 840-864 amino acids, 841-864 amino acids, 842-864 amino acids, 843-864 amino acids, 844-864 amino acids, 845-864 amino acids, 846-864 amino acids, 847-864 amino acids, 848-864 amino acids, 849-864 amino acids, 850-864 amino acids, 851-864 amino acid , amino acid 852-864, amino acid 853-864, amino acid 854-864, amino acid 855-864, amino acid 856-864, amino acid 857-864, amino acid 858-864, amino acids 859-864, amino acids 860-864, amino acids 862-864, or amino acids 863-864 of the tetanus toxin protein.

In some embodiments, the partial segment of the segment C of the tetanus toxin protein may comprise the amino acid sequence shown in SEQ ID NO:7.

In some embodiments, the partial segment of the segment C of the tetanus toxin protein may comprise the amino acid sequence shown in SEQ ID NO:8.

In some embodiments, the tetanus toxin protein may comprise at least about 70% (e.g., at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% , or at least about 100%) sequence identity to the amino acid sequence set forth in SEQ ID NO: 1-3.

In some embodiments, the protein variant may comprise at least about 70% (e.g., at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% , or at least about 100%) part of the segment C of the tetanus toxin protein.

In some embodiments, the protein variant comprises the amino acid sequence set forth in any one of SEQ ID NO: 1-2. In some embodiments, the protein variant comprises at least about 70% (e.g., at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% , or at least about 100%) sequence identity to the amino acid sequence set forth in any one of SEQ ID NO: 1-2.

In some embodiments, various additions, deletions and substitutions may be made to the protein variant amino acid sequence to generate the variant without adversely affecting the carrier capacity of the molecule. For example, conservative and semi-conservative amino acid substitutions can be made. Exemplary conservative amino acid substitutions include, but are not limited to, the following changes: alanine to serine; arginine to lysine; asparagine to glutamine or histidine; cysteine to serine; glutamine to asparagine; glutamate to aspartic acid; glycine to proline; histidine to asparagine or glutamine; isoleucine to leucine or valine; leucine to valine or isoleucine; lysine acids to arginine, glutamine, or glutamic acid; conversion of methionine to leucine or isoleucine; conversion of phenylalanine to tyrosine, leucine, or methionine; serine to threonine ; threonine to serine; tryptophan to tyrosine; tyrosine to tryptophan or phenylalanine; valine to isoleucine or leucine. As will be understood by one of ordinary skill in the art of protein engineering, the conservative and semi-conservative mutations mentioned herein do not affect the carrier function of the TTD. For example, suitable regions for mutation for various purposes (eg, adding a polypeptide "tag" to simplify purification of the protein) include C-terminal and N-terminal regions.

In another aspect, the present application provides a nucleic acid molecule comprising a protein variant encoding the tetanus toxin protein. In some embodiments, the TTD-encoding nucleic acid sequence comprises a wild-type TTD nucleic acid sequence, sequence segment, complementary sequence, and/or homolog thereof. In some embodiments, the TTD-encoding nucleic acid sequence comprises a mutation, truncation, substitution mutation, or translocation of a wild-type TTD nucleic acid sequence.

In some embodiments, the nucleic acid coding sequence expressed by the TTD protein is codon-optimized. For example, the nucleic acid coding sequence of the TTD protein is shown in any one of SEQ ID NO: 4-6. For example, the codon-optimized TTD nucleotide sequence can comprise a nucleotide sequence having at least about 70% (e.g., at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% , or at least about 100%) sequence identity to the nucleotide sequence shown in any one of SEQ ID NO: 4-6. In some embodiments, the nucleic acid coding sequence of the segment C of the tetanus toxin protein is codon-optimized. For example, the nucleic acid coding sequence of the segment C of the tetanus toxin protein is shown in SEQ ID NO: 6. For example, the segment C of the codon-optimized tetanus toxin protein can comprise a nucleotide sequence having at least about 70% (e.g., at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% %, or at least about 100%) sequence identity to the nucleotide sequence set forth in SEQ ID NO:6. The codon optimization can increase the protein expression of the TTD. For example, after the codon optimized nucleic acid sequence expressed by the TTD protein is introduced into cells, the protein expression level, sequence integrity, sequence accuracy and/or amplification are increased by at least about 10% (for example, at least about 15%, at least about 20%, at least about 30%, at least about 40%, at least about 50%, at least about 60%, at least about 70%, at least about 80%, at least about 90% or higher).

In some embodiments, the TTD protein may comprise the T cell epitope p2. In some embodiments, the T cell epitope p2 may comprise amino acids 830 to 844 of the tetanus toxin protein. In some embodiments, the TTD protein can comprise a nucleotide sequence that is at least about 70% (e.g., at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 100%) sequence identical to the amino acid sequence of the T cell epitope p2.

In some embodiments, the TTD protein can achieve high-yield protein expression by cloning its expression sequence into a protein expression vector. In some embodiments, the host of the protein expression vector can be an expression system commonly used in the art, for example, *Escherichia coli* expression system, *Pichia pastoris* expression system, or *Bacillus subtilis* expression system, insect cell expression system, plant cell expression system system and/or mammalian expression system. For example, the protein expression system may include an *E. coli* expression system.

In another aspect, the present application provides a vector which comprises the nucleic acid molecule. In some embodiments, the vector may comprise the nucleic acid molecule, an origin of replication, a selectable marker gene, a multiple cloning site, an enhancer, a promoter, and a terminator. There are many types of expression vectors, and any suitable expression vector can be used. Commonly used are plasmid expression vectors, cosmid vectors and viral vectors. In some embodiments, the vectors can include prokaryotic expression vectors and eukaryotic expression vectors, such as pET300, pET302, pBAD vectors of the E. *coli* expression system; derivatives of phage DNA, such as M 13 and other filamentous single-stranded DNA phages; pPIC9, pPIC9K, pPIC3K, pPICZ of Pichia expression systems; pGPST, pEB10, pEB20, pUB18 of *Bacillus subtilis* expression systems; SV40 of mammalian expression systems, adenovirus, well-known derivatives of retrovirus-derived DNA sequences and shuttle vectors derived from combinations of functional mammalian vectors, such as those described above, as well as functional plasmids and phage DNA. In some embodiments, the vector may comprise pGEX-4T, pET21.

In another aspect, the present application provides a cell, which may comprise the nucleic acid molecule and/or the vector. In some embodiments, the nucleic acid molecule may comprise a codon-optimized TTD nucleotide sequence. The cells described in this application can express the correct TTD protein. The cells may include cells from eukaryotes and prokaryotes, for example, *Saccharomyces cerevisiae, Pichia pastoris, Escherichia coli, Bacillus subtilis,* sf9, plant cells, CHO cells, HEK293 cells, COS cells, BHK cells, SP2/0 cells , NIH3T3 cells, etc. In the present application, the cells may comprise E. *coli* cells.

On the other aspect, the present application provides a carrier protein, which comprises the tetanus toxin protein variant and derivatives, homologues and analogs thereof. In some embodiments, the tetanus toxin protein variant may bind a polysaccharide from the bacterium or an oligosaccharide derived therefrom. In some embodiments, the bacterial polysaccharides include, but are not limited to, Streptococcus pneumoniae polysaccharides, T. *tularensis* endopolysaccharides, *Bacillus anthracis* polysaccharides, *Haemophilus influenzae* polysaccharides, *Salmonella typhi* polysaccharides, *Salmonella* species polysaccharides, and *Shigella* polysaccharides.

### Polysaccharide protein conjugate and preparation method thereof

In another aspect, the present application provides a polysaccharide protein conjugate, which may comprise the polysaccharide from Streptococcus pneumoniae and the truncated tetanus toxin protein. Most polysaccharides cannot induce thymus-dependent immune responses, and children under 2 years old or the elderly cannot acquire immunity, and therefore, it is necessary to chemically combine polysaccharides with protein carriers to make polysaccharides have thymus-dependent properties, induce relatively strong immunogenicity, and simultaneously induce anti-polysaccharide and anti-protein antibodies.

In some embodiments, the "polysaccharide from Streptococcus pneumoniae" may comprise a polysaccharide from the capsule of Streptococcus pneumoniae and an oligosaccharide derived from the polysaccharide. The Streptococcus pneumoniae can be from natural Streptococcus pneumoniae, or artificially modified Streptococcus pneumoniae. According to the antigenicity of capsular polysaccharides, Streptococcus pneumoniae can contain 91 serotypes, and the main pathogenic serotypes of pneumococcus can include the following 24 serotypes, which are 1, 2, 3, 4, 5, 6A, 6B, 7F, 8, 9N, 9V, 10A, 11A, 12F, 14, 15B, 17F, 18C, 19A, 19F, 20, 22F, 23F and 33F. In some embodiments, the polysaccharide of the polysaccharide protein conjugate has more than one serotype of Streptococcus pneumoniae, for example, at least 1, at least 2, at least 3, at least 4, at least 5, at least 6 , at least 7, at least 9, at least 12, at least 15, or at least 24.

In some embodiments, the polysaccharide of the polysaccharide protein conjugate can be selected from any serotype of Streptococcus pneumoniae in the following group: 1, 3, 5, 6A, 6B, 7F, 10A, 12F, 15B, 19A, 19F and 33F.

In the present application, the saccharides derived from the Streptococcus pneumoniae capsule may also include oligosaccharides derived from capsular polysaccharides. The "polysaccharide" generally refers to a polysaccharide high molecular carbohydrate composed of at least 10 monosaccharides. The "oligosaccharides" contain at least 2 saccharide residues. The capsular polysaccharide of Streptococcus pneumoniae can contain repeating oligosaccharide units of up to 8 saccharide residues. In some embodiments, the capsular saccharide that is the antigen can be a full-length polysaccharide, or it can be an oligosaccharide unit, or it can be a unit that is shorter than the natural length of the saccharide chain of the repeating oligosaccharide unit. In some embodiments, all saccharides present in the polysaccharide protein conjugate are polysaccharides.

In some embodiments, the polysaccharides and/or oligosaccharides may not be naturally occurring polysaccharides and/or oligosaccharides, they may be synthesized by any technique known in the art, and may be derived or modified from naturally occurring compounds. For example, full-length polysaccharides can be "sized", e.g. their size can be reduced by various methods, e.g. by acid hydrolysis treatment, peroxide treatment, by subsequent treatment with peroxide to generate oligosaccharide fragments to adjust their size. For example, modification of polysaccharides and/or oligosaccharides may include purification, carboxylation, sulfonation, sulfation, depolymerization and/or deacetylation.

In some embodiments, the modification of polysaccharides and/or oligosaccharides can be performed before their activation, or a post-treatment step can be used.

In some embodiments, the modified polysaccharide or oligosaccharide may have increased antigenicity compared to wild type.

In some embodiments, the polysaccharides and/or oligosaccharides described herein may be derived from antigen-binding fragments of the Streptococcus pneumoniae.

In some embodiments, the polysaccharides and/or oligosaccharides described herein can be synthesized from fragments of polysaccharides and/or oligosaccharides from different types and/or strains to form polysaccharides and/or oligosaccharides containing multiple epitopes.

In some embodiments, the Streptococcus pneumoniae polysaccharide can be prepared by techniques known in the art. For example, the prepared Streptococcus pneumoniae strains are amplified and cultured, sterilized and inactivated, the clarified culture solution is collected, a polysaccharide precipitation agent is added, and refined polysaccharides are obtained through purification steps. The purification step may include adding NaCl solution to dissolve the complex saccharide, centrifuging after dissolving, taking the supernatant, and adding alcohol to remove impurities and saccharide deposits. It can be washed and precipitated to remove impurities, and purified by chromatography such as ion exchange columns and composite packings to obtain refined polysaccharides.

In some embodiments, the truncated tetanus toxin protein may comprise the segment C of the tetanus toxin protein and the tetanus toxin protein variant.

In some embodiments, the truncated tetanus toxin protein comprises the segment C of the tetanus toxin protein.

In some embodiments, the truncated tetanus toxin protein may comprise the amino acid sequence shown in any one of SEQ ID NO: 1-3. The truncated tetanus toxin protein may comprise at least about 70% (e.g., at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% %, or at least about 100%) sequence identity to the amino acid sequence set forth in any one of SEQ ID NO: 1-3. In some embodiments, the nucleic acid coding sequence expressing the truncated tetanus toxin protein is obtained by codon optimization. For example, the nucleic acid coding sequence of the truncated tetanus toxin protein is shown in any one of SEQ ID NO: 4-6. For example, the nucleotide sequence of the codon-optimized truncated tetanus toxin protein can comprise the nucleotide sequence having at least about 70% (for example, at least about 75%, at least about 80%, at least about 85%, at least about 90%, at least about 91%, at least about 92%, at least about 93%, at least about 94%, at least about 95%, at least about 96%, A nucleotide sequence having at least about 97%, at least about 98%, at least about 99%, or at least about 100%) sequence identity with the nucleotide sequence shown in any one of SEQ ID NO: 4-6.

In some embodiments, the polysaccharide protein conjugate comprises the truncated tetanus toxin protein and the capsular polysaccharide or its derivative oligosaccharide from any one of the serotypes of Streptococcus pneumoniae. In some embodiments, the polysaccharide protein conjugate comprises the truncated tetanus toxin protein and the Streptococcus pneumoniae capsular polysaccharide or its derivative oligosaccharide from any serotype of Streptococcus pneumoniae selected from the group consisting of: 1, 2, 3, 4, 5, 6A, 6B, 7F, 8, 9N, 9V, 10A, 11A, 12F, 14, 15B, 17F, 18C, 19A, 19F, 20, 22F, 23F, and 33F. In some embodiments, the polysaccharide protein conjugate comprises the truncated tetanus toxin protein and the Streptococcus pneumoniae capsular polysaccharide or its derivative oligosaccharide from any serotype of Streptococcus pneumoniae selected from the group consisting of: 1, 3, 5, 6A, 6B, 7F, 10A, 12F, 15B, 19A, 19F, and 33F. In some embodiments, the polysaccharide protein conjugate comprises the segment C of the tetanus toxin protein and the Streptococcus pneumoniae capsular polysaccharide or its derivative oligosaccharide from any serotype of Streptococcus pneumoniae selected from the group consisting of: 1, 3, 5, 6A, 6B, 7F, 10A, 12F, 15B, 19A, 19F, and 33F. In some embodiments, the polysaccharide protein conjugate comprises the segment C of the tetanus toxin protein and the Streptococcus pneumoniae capsular polysaccharide or its derivative oligosaccharide from any serotype of Streptococcus pneumoniae selected from the group consisting of: 1, 3, 6A, and 15B. In some embodiments, the polysaccharide protein conjugate comprises the tetanus toxin protein variant TTD-1 and the Streptococcus pneumoniae capsular polysaccharide or its derivative oligosaccharide from any serotype of Streptococcus pneumoniae selected from the group consisting of: 3, 6B, 7F, and 19A. In some embodiments, the polysaccharide protein conjugate comprises the tetanus toxin protein variant TTD-2 and the Streptococcus pneumococcal serotype 10A capsular polysaccharide or its derivative oligosaccharide.

In the present application, the protein may be combined with at least 1 (for example, at least 2, at least 3, at least 4 or at least 5) of the polysaccharides, and the polysaccharides may contain 1 or more (for example, at least 2, at least 3 or at least 4) polysaccharides of Streptococcus pneumoniae serotypes. In the present application, the polysaccharide can be combined with more than one protein molecule (for example, at least 2, at least 3, at least 4 or at least 5).

In the present application, the "mass ratio" may include the ratio of the molecular mass of the polysaccharide to the molecular mass of the protein. In some embodiments, the mass ratio of the polysaccharide to the truncated tetanus toxin may be 0.4-2.5.

In some embodiments, the mass ratio of the Streptococcus pneumoniae serotype 3 polysaccharide to the truncated protein may be 0.4-2.5.

In some embodiments, the mass ratio of the Streptococcus pneumoniae serotype 5 polysaccharide to the truncated protein may be 0.4-2.5.

In some embodiments, the mass ratio of the Streptococcus pneumoniae serotype 6A polysaccharide to the truncated protein may be 0.4-2.5.

In some embodiments, the mass ratio of the Streptococcus pneumoniae serotype 6B polysaccharide to the truncated protein may be 0.4-2.5.

In some embodiments, the mass ratio of the Streptococcus pneumoniae serotype 12F polysaccharide to the truncated protein may be 0.4-2.5.

In some embodiments, the mass ratio of the Streptococcus pneumoniae serotype 15B polysaccharide to the truncated protein may be 0.4-2.5.

In some embodiments, the mass ratio of the Streptococcus pneumoniae serotype 19A polysaccharide to the truncated protein may be 0.4-2.5.

In some embodiments, the mass ratio of the Streptococcus pneumoniae serotype 19F polysaccharide to the truncated protein may be 0.4-2.5.

In some embodiments, the mass ratio of the Streptococcus pneumoniae serotype 33F polysaccharide to the truncated protein may be 0.4-2.5.

In this application, the polysaccharide protein conjugate can be prepared by any known conjugation technique. Conjugation methods can rely on the activation of saccharides with 1-cyano-4-dimethylaminopyridine tetrafluoroborate (CDAP) to form cyanate esters. The activated saccharides can thus be conjugated to an amino group on the carrier protein either directly or through a spacer (linker) group. For example, the spacer can be cystamine or cysteamine to generate thiolated polysaccharides, and the latter can be conjugated to the carrier via a thioether bond obtained after reaction with a maleimide-activated carrier protein (e.g. using GMBS) or with a haloacetylated carrier protein (for example with iodoacetimide (e.g. ethyl iodoacetimide) or N-succinimidyl bromoacetate or SIAB, or SIA, or SBAP). In some embodiments, depending on the characteristics of the polysaccharide, cyanogen bromide method (US5952454), 1-cyano-4-dimethylaminopyridine tetrafluoroborate (CDAP) (EP0720485), periodic acid oxidation method (US4711779) can be chosen.

### Methods of improving immunogenicity

In another aspect, the present application provides a method for improving immunogenicity, which may include the following steps: providing a polysaccharide protein conjugate comprising the polysaccharide from Streptococcus pneumoniae and the truncated tetanus toxin. Most polysaccharides belong to T cell-independent type 2 (TI-2), and after entering the body, they can activate B cells by crossing-linking with the antigen recognition receptors on the surface of B cells, namely surface immunoglobulin molecules (sIg), which causes a series of downstream changes and makes B cells differentiate into antibody-secreting plasma cells, and no T cells participate in the entire immune process, and therefore no immune memory is formed, and the antibodies produced are mainly IgM and IgG2 with lower affinity. After binding to glycoproteins, polysaccharide protein conjugates can be presented by antibody-presenting cells, and mature B cells are activated by T helper cells to induce IgG-like antibodies, which can cause immune memory responses. Therefore, the type of carrier protein has an impact on the immunogenicity of polysaccharide conjugates. The most typical case is the withdrawal of the Hib conjugate vaccine with DT as the protein carrier: compared with tetanus toxoid (TT), CRM197 and other protein-carrier conjugate vaccines, the Hib conjugate vaccine with DT as the carrier has poor immunogenicity in infants under 18 months, so it was eliminated after launch; in addition, a clinical study of a foreign 11-valent pneumonia conjugate vaccine showed that when coimmunized with DPT vaccine, the serum antibody against polysaccharide with TT as the carrier was significantly inhibited, and the study was discontinued due to this result, but this inhibitory effect was not present in the DT-carrier conjugate.

Currently, the carrier proteins used in marketed conjugate vaccines include diphtheria toxoid (DT), diphtheria toxoid mutant (CRM197), tetanus toxoid (TT), Meningococcal B outer membrane protein complex (OMP), nontypeable Haemophilus influenzae protein D (PD). Carrier proteins currently being studied and developed include recombinant Pseudomonas aeruginosa exotoxin A (rEPA), recombinant Staphylococcus aureus enterotoxin C1 (rSEC), cholera toxin B subunit (CTB), etc. The avirulent variant of diphtheria toxin (CRM197) is a commonly used carrier protein that has been clinically proven to be safe and effective, and has been widely used in marketed pneumococcal polysaccharide conjugate vaccines. CRM197 is produced by C diphtheriae infected with the non-toxigenic phage β197tox produced by nitrosoguanidine mutagenesis of the toxigenic carynephage b (Uchida et al. Nature New Biology (1971) 233; 8-11). The CRM197 protein has a similar sequence and molecular weight to diphtheria toxin, but differs from diphtheria toxin in a single base change in the structural gene. This results in a change of amino acid position 52 from glycine to glutamine, which renders the fragment unable to bind NAD and thus not toxic (Pappenheimer 1977, Ann Rev, Biochem. 46; 69-94, Rappuoli Applied and Environmental Microbiology Sept 1983p560 -564).

The 13-valent pneumococcal conjugate vaccine Prevenar-13 developed by Pfizer used CRM197 (a non-toxic variant of diphtheria toxoid) as the carrier protein and was first launched in the United States in 2010, and yet the immunogenicity of polysaccharides of different serotypes was found in clinical applications relatively large difference. The comparison between TTD as a new carrier protein and CRM197 has important clinical significance and value. In the present application, the improvement of the immunogenicity means that the polysaccharide has the function of improving the immunogenicity of the polysaccharide after binding to the truncated tetanus toxin protein. In some embodiments, the improvement of immunogenicity comprises a conjugate of the polysaccharide and the truncated tetanus toxin protein being no less or more immunogenic than a conjugate of the polysaccharide CRM197.

In the present application, the immunogenicity can be detected by any method known to those skilled in the art. In some embodiments, the detection method may include the following steps: the polysaccharide-truncation conjugate and the polysaccharide-CRM197 conjugate are respectively added to an optional adjuvant to prepare an immune antigen; the immune antigen can be introduced into the body of the subject, and the blood sample of the subject is drawn for detection within a certain period of time. In the present application, the adjuvant may include aluminum hydroxide gel, aluminum phosphate or aluminum salts of alum, but may also be other metal salts such as salts of calcium, magnesium, iron or zinc; or may be acyl tyrosine, or acylated saccharides, cationically or anionically derivatized saccharides, or insoluble mixtures containing polyphosphazenes. In some embodiments, the adjuvant may include aluminum hydroxide, aluminum phosphate, and/or Freund's adjuvant. In some embodiments, the introducing may include introducing through intravenous injection, intraperitoneal injection, intramuscular injection, subcutaneous injection, etc.

In some embodiments, the detection methods can comprise detection of antibody titer in immune serum by ELISA-bridge method, direct ELISA method, indirect ELISA method, radioimmunoassay, electrochemiluminescence, surface plasmon resonance, enzyme-linked immunospot method, immuno-PCR method. In some embodiments, the detection methods may comprise detecting the production of specific antibodies by an opsonophagocytic killing assay.

### Pharmaceutical composition, vaccine, use and method of prevention

In another aspect, the present application provides a pharmaceutical composition comprising the polysaccharide protein conjugate and optionally a pharmaceutically acceptable adjuvant. The suitable adjuvant may include aluminum salts such as aluminum hydroxide gel, aluminum phosphate or alum, but also other metal salts such as salts of calcium, magnesium, iron or zinc; it may alternatively be an acylated tyrosine, or an acylated saccharide, a cationically or anionically derivatized saccharides, or an insoluble mixture containing polyphosphazene.

In another aspect, the present application provides the use of the tetanus toxin protein variant for preparing medicine. In some embodiments, the use may include the treatment of bacterial diseases, such as diseases caused by Gram-positive bacteria, such as *Staphylococcus aureus, Staphylococcus epidermidis, a-hemolytic streptococcus, β-hemolytic streptococcus, non-hemolytic streptococcus, pneumococcus, enterococcus* and others. In some embodiments, the use may include the treatment of diseases caused by Gram-negative bacteria, such as *meningococcus, gonorrhoeae, morakata, Acinetobacter, Pseudomonas, Alcaligenes faecalis, Brucella, Pertussis, Legionella, Salmonella, Shigella, Klebsiella.* In some embodiments, the bacterial disease may comprise Streptococcus pneumoniae disease. In some embodiments, the Streptococcus pneumoniae disease can include pneumonia, sepsis, meningitis, and/or otitis media.

In another aspect, the present application provides use of the polysaccharide conjugate for the preparation of medicaments. In some embodiments, the use may involve the prevention and/or treatment of bacterial diseases. In some embodiments, the bacterial disease may comprise Streptococcus pneumoniae disease. In some embodiments, the Streptococcus pneumoniae disease can include pneumonia, sepsis, meningitis, and/or otitis media.

In this application, the "prevention" generally refers to preventing the occurrence and onset, recurrence, and/or spread of a disease or one or more symptoms thereof by taking certain measures in advance. The "treatment" generally refers to eliminating or improving a disease, or alleviating one or more symptoms associated with a disease. For example, using the tetanus toxin protein variant herein and/or the medicament prepared thereof prevents the occurrence, onset, recurrence and/or spread of bacterial diseases. For example, the use of the polysaccharide conjugates described herein and/or the medicament prepared thereof prevents the occurrence, onset, recurrence and/or spread of bacterial diseases. For example, the bacterial disease may be Streptococcus pneumoniae disease. For example, the Streptococcus pneumoniae disease can include pneumonia, sepsis, meningitis and/or otitis media.

In another aspect, the present application provides a vaccine which comprises the polysaccharide protein conjugate, the pharmaceutical composition and optionally a pharmaceutically acceptable adjuvant. The polysaccharide protein conjugate may comprise the truncated tetanus toxin protein, the tetanus toxin protein variant and/or the segment C of the tetanus toxin protein. Each type of carrier protein can serve as a carrier for more than one saccharide, where the saccharides can be the same or different. For example, the serotypes 3 and 5 of Streptococcus pneumoniae can be conjugated to the same carrier protein, or bound to the same molecule of the carrier protein, or to different molecules of the same carrier protein. In some embodiments, two or more different saccharides can be bound to the same carrier protein, either to the same molecule of a carrier protein, or to different molecules of the same carrier protein.

Vaccine formulations containing the pharmaceutical compositions of the present application can be administered by systemic or mucosal routes for the protection or treatment of mammals susceptible to infection. These administrations may include injection by intramuscular, intraperitoneal, intradermal or subcutaneous routes; or mucosal administration to the oral/digestive, respiratory, genitourinary tracts. In some embodiments, the components of the pharmaceutical composition may also be co-administered at the same time or at different times (for example, the Streptococcus pneumoniae glycoconjugate can be administered separately at the same time or 1-2 weeks after the administration of any bacterial protein component of the vaccine, so that the immune response between the two can be optimally matched). For coadministration, optional adjuvants may be present in any or all of the different administrations. Instead of a single route of administration, 2 different routes of administration can also be used. For example, saccharides or saccharide conjugates can be administered intramuscularly (or intradermally), while bacterial proteins can be administered mucosally (or intradermally).

In another aspect, the present application provides a method for preventing Streptococcus pneumoniae disease which may comprise administering the polysaccharide protein conjugate, the nucleic acid molecule, the vector, the cell, the carrier protein, the pharmaceutical composition and/or the vaccine to a subject in need. In some embodiments, the Streptococcus pneumoniae disease can include pneumonia, sepsis, meningitis, and/or otitis media.

In some embodiments, the polysaccharide protein conjugate, the pharmaceutical composition and/or the pharmaceutically acceptable adjuvant provided in this application can be used to prepare antiserum. In some embodiments, the polysaccharide protein conjugate, the pharmaceutical composition and/or the pharmaceutically acceptable adjuvant provided in this application can be used to prepare antibodies. The polysaccharide protein conjugate may comprise the truncated tetanus toxin protein, the tetanus toxin protein variant and/or the segment C of the tetanus toxin protein. Each type of carrier protein can serve as a carrier for more than one saccharide, where the saccharides can be the same or different. The saccharide may be the Streptococcus pneumoniae capsular polysaccharide.

In some embodiments, the polysaccharide protein conjugate, the pharmaceutical composition and/or the pharmaceutically acceptable adjuvant provided herein can be used to detect antibodies in a sample. In some embodiments, the polysaccharide protein conjugate, the pharmaceutical composition and/or the pharmaceutically acceptable adjuvant provided herein can be used to prepare a kit for detecting antibodies in a sample.

For example, the antibody may be an antibody of bacteria. For example, the antibody of bacteria may be an antibody to Streptococcus pneumoniae. For example, antibodies to the Streptococcus pneumoniae serotype 3, antibodies to the Streptococcus pneumoniae serotype 5, antibodies to the Streptococcus pneumoniae serotype 6A, antibodies to the Streptococcus pneumoniae serotype 6B, antibodies to the Streptococcus pneumoniae serotype 10A, antibodies to the Streptococcus pneumoniae serotype 12F, antibodies to the Streptococcus pneumoniae serotype 15B, antibodies to the Streptococcus pneumoniae serotype 19A, and/or antibodies to the Streptococcus pneumococcal serotype 33F.

In some embodiments, the tetanus toxin protein variant, the truncated tetanus toxin protein and/or the segment C of the tetanus toxin protein provided by the application can also be used for preparing antibodies against the tetanus toxin, for preparing antigens and/or vaccines against the tetanus toxin, and/or for preparing a kit for diagnosing antibodies against the tetanus toxin.

In another aspect, the present application provides use of the polysaccharide conjugate as an antigen for the preparation of antibodies.

In certain embodiments, the antibody is used for diagnostic typing of isolated bacterial strains.

In another aspect, the present application provides a kit which comprises the polysaccharide conjugate.

In certain embodiments, the kit is used for is used for diagnostic typing of isolated bacterial strains.

Without be limited by any theory, the following examples are only for explaining the protein variants, preparation methods and uses of the present application, and are not intended to limit the scope of the present invention.

### Example

### Example 1: design of TTD recombinant protein sequence

Based on the published tetanus neurotoxin sequence (NBCI WP011100836) and its crystal structure (PDB: SNOB) of Clostridium tetani, this application designs a truncated tetanus toxin protein (Tetanus Toxin Domain, TTD) that excludes the N-terminal sequence of the tetanus toxin protein (Tetanus toxin, TT), and contains its C-terminal sequence. Therefore, the TTD protein does not have the toxicity of the tetanus toxin protein, but retains its C-terminal domain and functional fragments, and while maintaining the correct folding and stability of the protein, it provides T cell epitopes and structural flexibility, which is more conducive to chemical conjugation.

The designed protein sequence 01 (TTD-1) is a TTD protein sequence containing 487 amino acids obtained by removing 828 amino acids at the N-terminus of the TT sequence, i.e., the N-terminal peptidase M27 and part of the translocation region are removed, and the C-terminal functional domain (receptor binding domain) is retained, and the amino acid sequence is shown in SEQ ID NO: 1. The corresponding nucleic acid sequence (TTD-4) is codon-optimized as shown in the sequence SEQ ID NO: 4.

The designed protein sequence 02 (TTD-2) is a TTD protein sequence containing 476 amino acids obtained by removing 839 amino acids at the N-terminus of the TT sequence, i.e., the N-terminal peptidase M27 and part of the translocation region are removed, and the C-terminal functional domain (receptor binding domain) is retained, and the amino acid sequence is shown in SEQ ID NO: 2. The corresponding nucleic acid sequence (TTD-5) is codon-optimized as shown in the sequence SEQ ID NO: 5.

The designed protein sequence 03 (TTD-3) is a TTD protein sequence containing 451 amino acids obtained by removing 864 amino acids at the N-terminus of the TT sequence, i.e., the N-terminal peptidase M27 and part of the translocation region are removed, and the C-terminal functional domain (receptor binding domain) is retained, and the amino acid sequence is shown in SEQ ID NO: 3. The corresponding nucleic acid sequence (TTD-6) is codon-optimized as shown in the sequence SEQ ID NO: 6.

### Example 2: expression vector construction and protein expression

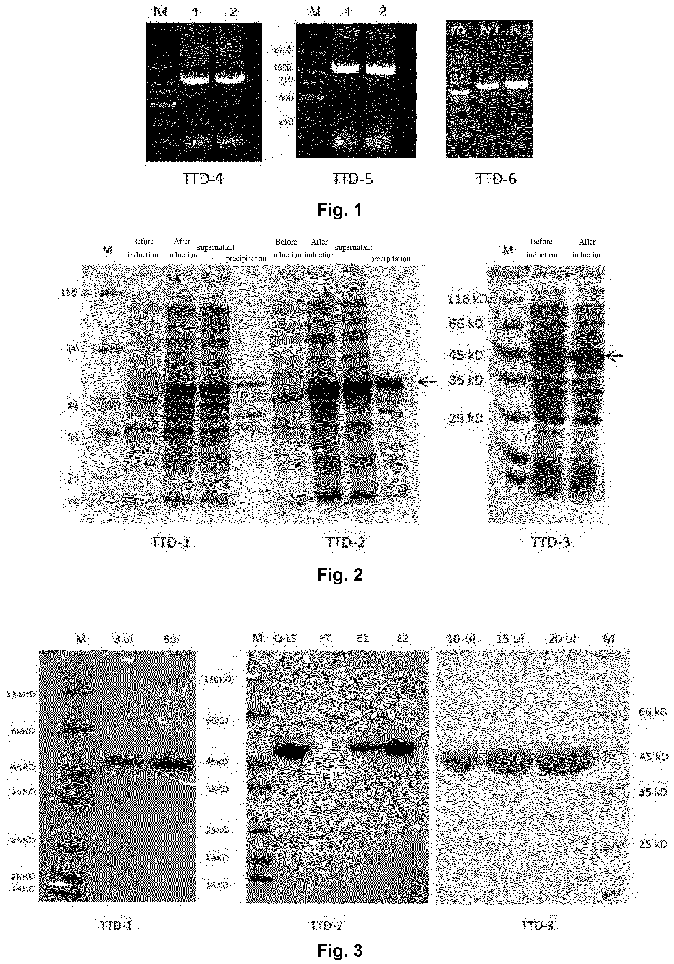

In order to utilize the *E. coli* protein expression system, the designed protein TTD and its corresponding gene sequence are successively constructed into protein expression vectors (such as pGEX-4T, pET21) to obtain the recombinant expression plasmid. After the recombinant protein expression plasmid is transformed into BL21(DE3) competent cells (Thermo Fisher), the recombinant expression plasmid is obtained. The PCR electrophoresis results of TTD-4, TTD-5 and TTD-6 are shown in Figure 1. The preserved strains on the plate containing Amp⁺ are streaked and activated, and a single clone is picked, inoculated into the LB liquid medium containing Amp⁺, and cultured overnight at 37°C and 250rpm. The cultured strains are diluted in proportion and inoculated in LB liquid medium containing Amp⁺, cultured with shaking at 37°C until the OD600 reaches 0.5-1.5, and then induced by adding 1 mM IPTG. After the culture, 1 mL of the sample is taken and centrifuged, the supernatant is removed and the lysate is added for SDS-PAGE electrophoresis analysis to determine the expression of the recombinant protein. The electrophoresis results are shown in Figure 2, and the target TTD protein expression is thus confirmed.

### Example 3: purification of TTD protein

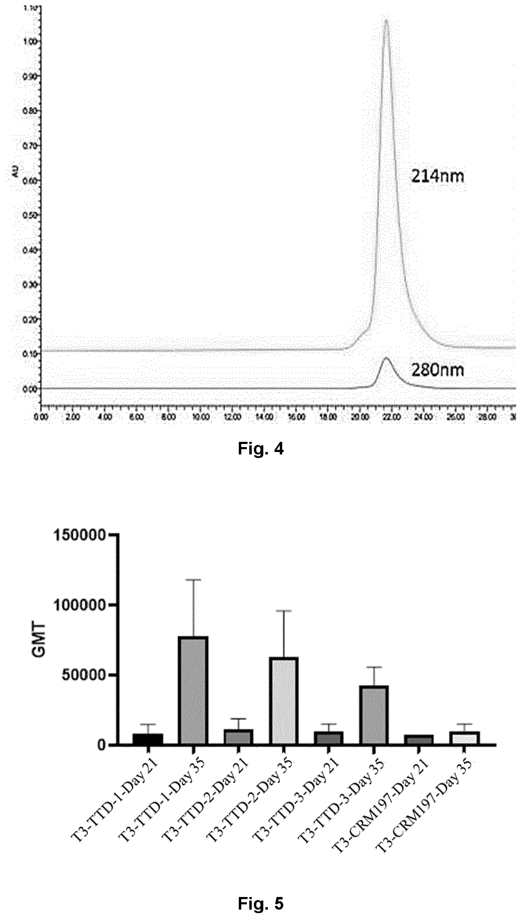

25 g of recombinant TTD bacterial cells obtained by fermentation are dissolved with 10 times 50 mM Tris-HCl buffer solution to form a 250 mL system, and crushed twice with a high-pressure homogenizer, and the precipitate is removed after high-speed centrifugation, and the supernatant is kept for use. Ammonium sulfate is added to the supernatant to precipitate, stirred at low temperature for 2 hours, and then the precipitate is collected by high-speed centrifugation for 1 hour, and the collected precipitate is dissolved with buffer, changed the liquid by ultrafiltration, and then passed through mixed mode chromatography and ion-exchange chromatography respectively, and different NaCl concentration gradient elution are used to remove impurities and finally TTD protein with a purity of more than 95% is obtained. The purification results are shown in Figure 3. SEC-HPLC analysis of the purified TTD protein showed that the protein had good homogeneity. The SEC-HPLC results of TTD-1 are shown in Figure 4.

### Example 4: preparation of Streptococcus pneumoniae polysaccharide

A tube of prepared Streptococcus pneumoniae strain (ATCC) is inoculated into a shaker flask, and cultured overnight in a 37°C CO₂ incubator. After the microscopic examination showing normal result, the cultured solution is inoculated into a 10-liter fermenter and fermented for 6-10 hours. After the cultivation, an inactivator is added to inactivate the bacteria, then the clarified culture fluid is collected by centrifugation, and a polysaccharide precipitation agent is added to the clarified culture solution to obtain crude precipitated saccharide complex. After a series of purification steps, refined polysaccharides are obtained. Usually, the refined polysaccharide purification process includes, but is not limited to, adding NaCl solution to dissolve the saccharide complex, centrifuging after dissolving, taking the supernatant, adding alcohol to remove impurities and saccharide precipitation. It can be washed and precipitated to remove impurities, and purified by chromatography such as ion exchange or mixed mode chromatography to obtain refined polysaccharides. The purified polysaccharides of different serotypes passed all quality control analysis, and all analytical data meet the criteria based on the standards from the European Pharmacopoeia, for example, the content of impurities such as protein and nucleic acid is less than 1%.

### Example 5: preparation of Streptococcus pneumoniae polysaccharide-protein conjugates

Streptococcus pneumoniae polysaccharide has chemical reactivity with carrier protein after activation. The polysaccharide-protein chemical conjugation can be conjugated directly or through a linker, such as linker adipate dihydrazide conjugation. The common methods of chemical conjugation are through cyanogen bromide method, CDAP method or reductive amination method (US5952454, EP0720485, US4711779). The polysaccharide-protein conjugate obtained by conjugation can induce relatively strong immunogenicity, and can simultaneously induce specific antibodies against polysaccharides and proteins.

The steps for preparing bacterial polysaccharide-protein conjugates using the cyanogen bromide method are as follows: dissolving 200 mg of purified polysaccharide in 20 ml of 0.9% NaCl solution, adding cyanogen bromide, adding 0.5N NaOH solution, adjusting the pH of the solution to 10.5, and after reacting 10-15 min, adjusting the solution to pH 7.8 by adding 0.5 N HCl. Then adding adipate dihydrazide solution (ADH) to prepare polysaccharide-ADH derivatives. At the same time, the carrier protein (TTD or CRM97) is reacted with EDC (1-ethyl-3-(3-dimethylaminopropyl)-carbodimide hydrochloride) to prepare carrier protein derivatives. Finally, mixing the polysaccharide-ADH derivative and the activated carrier protein in proportion to react to form a polysaccharide-protein conjugate, and then concentrating by ultrafiltration or chromatography to remove impurities and unreacted substances, and obtaining a polysaccharide-protein conjugate.

The procedure for preparing bacterial polysaccharide-protein conjugates using the CDAP method is as follows: In a typical reaction, dissolving the pneumonia polysaccharide (50 mg) in 10 ml of sodium borate buffer (100 mM, pH 9.0), adding the CDAP solution, and 0.2 N NaOH solution subsequently to adjust the pH of the reaction solution to 9.0. After strring and reacting at room temperature for 5-10 minutes, adding 0.1 N HCl solution to adjust the pH to 7.5. Then desalting the reaction solution through a G25 column to collect activated polysaccharides. At the same time, dissolving the carrier protein in 0.1 M NaHCOs pH8.0 buffer, then mixing with the activated polysaccharide, and stirring for 6-10 hours at room temperature. After the conjugation reaction, concentrating and replacing the solution by ultrafiltration to remove impurities and finally obtaining the polysaccharide-protein conjugate.

The procedure for preparing bacterial polysaccharide-protein conjugates using the reductive amination method is as follows: In a typical reaction, dissolving the lyophilized polysaccharide in phosphate buffer (4 mg/ml), adding with 100 mM sodium periodate solution, and after stirring overnight, desalting through a G25 column to obtain activated polysaccharides. At the same time, dissolving the carrier protein in a phosphate buffer, then mixing it with the activated polysaccharide solution in proportion, adding a certain amount of sodium cyanoborohydride, and stirring overnight at room temperature or 37°C. After the reaction, chromatographing the reaction solution to remove impurities and purifying to obtain polysaccharide-protein conjugates.

### Example 6-15: immunogenicity evaluation of polysaccharide protein conjugates

Mouse immunization experiment protocol: adding the polysaccharide-protein conjugate of Streptococcus pneumoniae to aluminum hydroxide or aluminum phosphate adjuvant to prepare immune antigen, and selecting Balb/c mice aged 6-8 weeks for intraperitoneal immunization, with 5-8 mice in each group and a dose of 2 micrograms for each immunization, and performing immunizing on Day 0, Day 14, and Day 21 respectively, and drawing blood to evaluate the immunogenicity of the polysaccharide on Day 21 and Day 35.

Rabbit immunization experiment protocol: adding polysaccharide-protein conjugates to Freund's adjuvant to prepare immune antigens, and selecting 2~2.5kg New Zealand white rabbits for immunization, with 2 rabbits in each group. For the first time, emulsifying and mixing Freund's complete adjuvant with the antigen, with a dose of 200 micrograms for subcutaneous immunization; on Day 14, emulsifying and mixing Freund's incomplete adjuvant with the antigen, and performing the second subcutaneous immunization with a dose of 200 micrograms; on Day 28, administering 100 µg antigen for intravenous immunization. And on Day 21and Day 42, drawing blood to evaluate the immunogenicity of the polysaccharide.

The ELISA method to evaluate the immunogenicity of Streptococcus pneumoniae polysaccharide: diluting the Streptococcus pneumoniae polysaccharide with coating buffer, coating it on a 96-well microtiter plate, with 100 µl/well, and washing the plate after incubating at 37°C for 5 hours. After treating the mouse and rabbit antiserum with the adsorbent, firstly diluting the mouse serum at 1:200 (diluting the rabbit serum at 1: 10000), and then diluting it to 8 gradients for 2.5 fold each, and then adding it to the plate with 50 µl per well, and incubating it overnight. After washing the plate, diluting the secondary antibody at 1:20000 and adding it to the ELISA plate with 100 µl per well, and washing the plate after incubating for 2 hours, then adding 1 mg/ml PNPP-Na chromogenic substrate with 100 µl per well, and after incubating for 2 hours, adding 50 µl/well of 3M NaOH to stop the reaction, and recording the absorbance at a wavelength of 405 nm. If the ratio of the OD value of the test well to the OD value of the negative well is greater than or equal to 2.1, it is judged as positive, and the antibody titer of each serum is defined as its maximum dilution fold of the serum that shows positive reaction in ELISA. The geometric mean of the antibody titer of each group of immunized animals is calculated, using T-test to analyze the statistical significance among different groups.

The opsonophagocytic killing assay of Streptococcus pneumoniae capsular polysaccharide-specific antibody: diluting Streptococcus pneumoniae to 10⁵ CFU/ml, and adding it to 96-well cell working plate with 10 µl/well. Adding the inactivated serum sample to the above-mentioned cell working plate with 20 µl/well after gradient dilution, incubating the bacteria and antiserum at 700 rpm/min for 30 min, and washing the HL-60 cells differentiated by DMF with HBSS buffer and adjusting to a concentration of 1×10⁷ cells/ml, and then mixing the 1×10⁷ cells/ml cell solution with the diluted complement at a volume ratio of 1:4, and adding mixed solution to 96-well cell working plate with 50 µl/well. Putting the 96-well cell working plate on a mixer, putting it into a CO₂ incubator with 5% CO₂ and a temperature of 37°C, and incubating with shaking for 45 minutes. After the opsonophagocytosis was stopped, spotting the sample on a blood plate and culturing overnight in a CO₂ incubator. Calculating the sterilization rate of each diluted serum.

### Example 6: Immunoenhancing effect of carrier protein TTD on the polysaccharides of the serotype 3

By comparing the immune effect of the Streptococcus pneumoniae serotype 3 (T3) polysaccharide-TTD conjugate with the Streptococcus pneumoniae serotype 3 polysaccharide-CRM197, the immune enhancement effect of this serotype of Streptococcus pneumoniae polysaccharide-TTD in mice was analyzed.

Mice were immunized with the polysaccharides of the serotype 3-CRM197 conjugates and the serotype-3-polysaccharide-TTD-1, 2, 3 conjugates, and the antibody titers were evaluated on Day 21 and Day 35, respectively. The results are shown in Figure 5, on Day 35, T3-TTD-1, T3-TTD-2, and T3-TTD-3 groups can all induce higher antibody titers.

The antibody titer of the 35-day immune serum was analyzed between groups, and after ANOVA one-way analysis of variance, the comparison between T3-TTD group and T3-CRM197 group, P=0.001<0.01, the statistical difference was highly statistically significant, indicating that TTD-1, TTD-2 and TTD-3 carrier proteins all have more obvious immunoenhancing effects than CRM197.

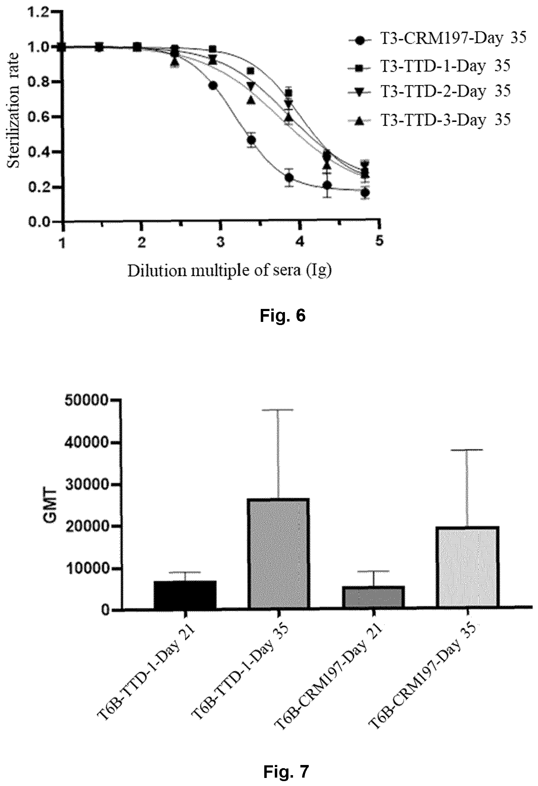

The immune sera of the two groups of T3-CRM197 and T3-TTD-1 on Day 35 were subjected to OPA test. The results are shown in Figure 6, the IC₅₀ (dilution fold of antiserum at 50% sterilization rate) of T3-CRM197 and T3-TTD-1, T3-TTD-2 and T3-TTD-3 were 1599 and 9926, 7142 and 5728. The results showed that TTD-1, TTD-2 and TTD-3 carrier protein conjugates induced significantly more specific antibodies against the serotype-3-polysaccharide than CRM197 carrier protein conjugates.

### Example 7: immunoenhancing effect of carrier protein TTD on the pneumococcal serotype 6B polysaccharide

By comparing the immune effect of the Streptococcus pneumoniae serotype 6B (T6B) polysaccharide-TTD-1 conjugate with the Streptococcus pneumoniae serotype 6B polysaccharide-CRM197, the immune enhancement effect of this serotype of Streptococcus pneumoniae polysaccharide-TTD-1 in mice was analyzed.

The mice were immunized with the serotype-6B-polysaccharide-CRM197 conjugate and the serotype-6B-polysaccharide-TTD-1 conjugate, and the antibody titers on Day 21 and Day 35 were evaluated respectively. The results are shown in Figure 7, on Day 35, the serotype-6B-polysaccharide-TTD-1 conjugate induce higher antibody titers.

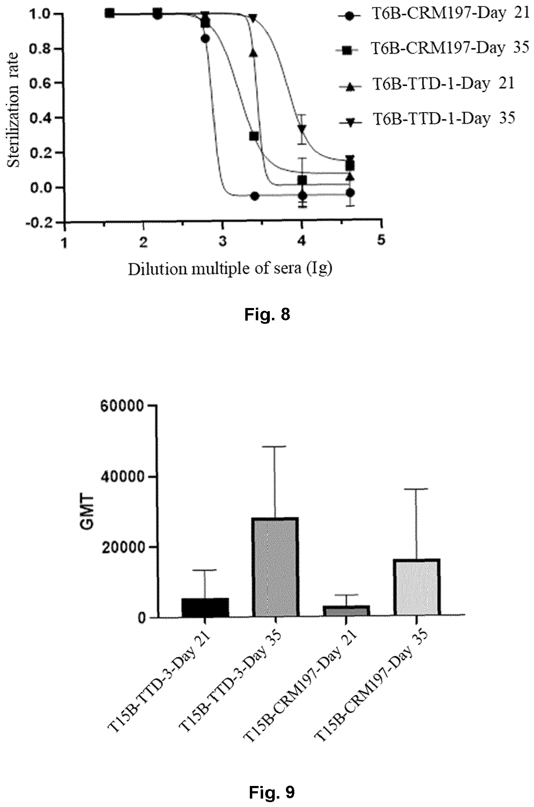

Immune sera of the two groups of the 6B-TTD-1 and 6B-CRM197 on Day 21 and Day 35 were subjected to OPA test. The results are shown in Figure 8, the IC₅₀ (dilution multiple of antiserum at 50% sterilization rate) of immunization serum of 6B-TTD-1 and 6B-CRM197 on Day 21 were 2851 and 746.3, respectively, and the IC₅₀ (dilution multiple of antiserum at 50% sterilization rate) of 6B-TTD-1 and 6B-CRM197 immunized sera on Day 35 were 7677 and 1802, respectively, indicating that the TTD-1 carrier protein conjugate was capable of inducing more specific functional antibodies against the serotype 6B polysaccharides than the CRM197 carrier protein conjugate.

### Example 8: immunoenhancing effect of carrier protein TTD on the pneumococcal serotype 15B polysaccharide

By comparing the immune effect of the Streptococcus pneumoniae serotype 15B (T15B) polysaccharide-TTD-3 conjugate with the of Streptococcus pneumoniae serotype 15B polysaccharide-CRM197, the immune enhancement effect of this serotype of Streptococcus pneumoniae polysaccharide-TTD in mice was analyzed.

Mice were immunized with the serotype 15B polysaccharide-CRM197 conjugates and the serotype 15B polysaccharide-TTD-3 conjugates, and the antibody titers were evaluated on Day 21 and Day 35, respectively, and the results are shown in Figure 9, the polysaccharide-TTD-3 conjugate can induce higher antibody titers.

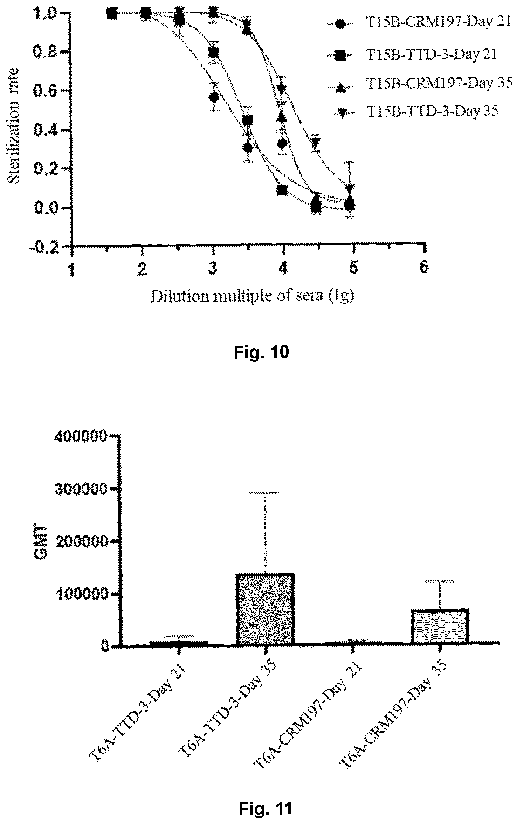

Immune sera of two groups of 15B-CRM197 and 15B-TTD-3 on Day 21 and Day 35 were subjected to OPA test. The results are shown in Figure 10, the IC₅₀ of the immune sera of 5B-CRM197 and 15B-TTD-3 on Day 21 are 1392 and 2633, respectively. The IC₅₀ of the immune sera of 5B-CRM197 and 15B-TTD-3 on Day 35 are 9005 and 14997, respectively. It indicates that TTD-3 carrier protein conjugates induce significantly more specific antibodies against the serotype 15B polysaccharides than CRM197 carrier protein conjugates.

### Example 9: immunoenhancing effect of carrier protein TTD on the pneumococcal serotype 6A polysaccharide

Mice were immunized with the Streptococcus pneumoniae serotype 6A (T6A) polysaccharide-CRM197 conjugate and the serotype-6A-polysaccharide-TTD-3 conjugate, and the antibody titers on Day 21 and Day 35 were evaluated respectively (as shown in Figure 11). On Day 35, the polysaccharide-TTD conjugate induce antibody titers not lower than polysaccharide-CRM197.

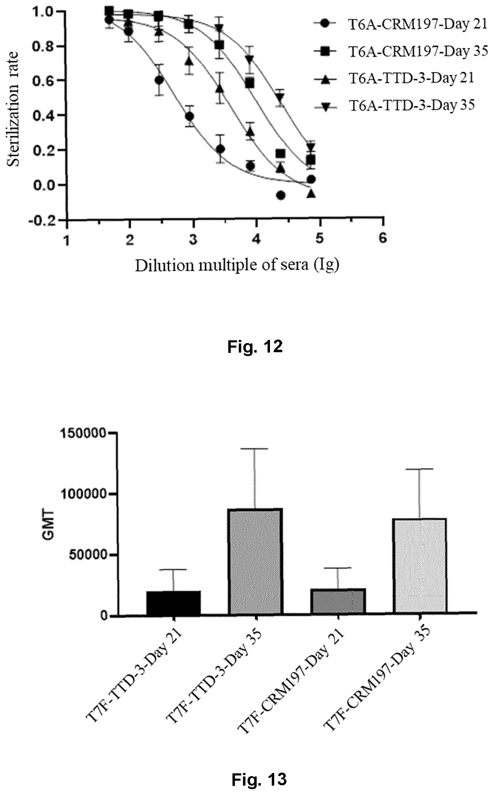

Immune sera of the two groups of T6A-CRM197 and T6A-TTD-3 on Day 21 and Day 35 were subjected to OPA test. The results are shown in Figure 12, the IC₅₀ of immune sera of T6A-CRM197 and T6A-TTD-3 on Day 21 are 458 and 1606, respectively. The IC₅₀ of immune sera of T6A-CRM197 and T6A-TTD-3 on Day 35 are 9103 and 20850, respectively, and TTD-3 carrier protein conjugates induce significantly more specific antibodies against the serotype 6A polysaccharides than CRM197 carrier protein conjugates.

### Example 10: immunoenhancing effect of carrier protein TTD on the pneumococcal serotype 7F polysaccharide

Provided are comparisons of the Streptococcus pneumoniae serotype 7F (T7F) polysaccharide-TTD-1 conjugates with polysaccharide-CRM197 conjugates. Mice were immunized with polysaccharide-CRM197 conjugates and polysaccharide-TTD-1 conjugates, and the antibody titers were evaluated on Day 21 and Day 35, respectively. The results are shown in Figure 13, on Day 35, the serotype-7F-polysaccharide-TTD-1 conjugate can induce equivalent antibody titers to polysaccharide-CRM197.

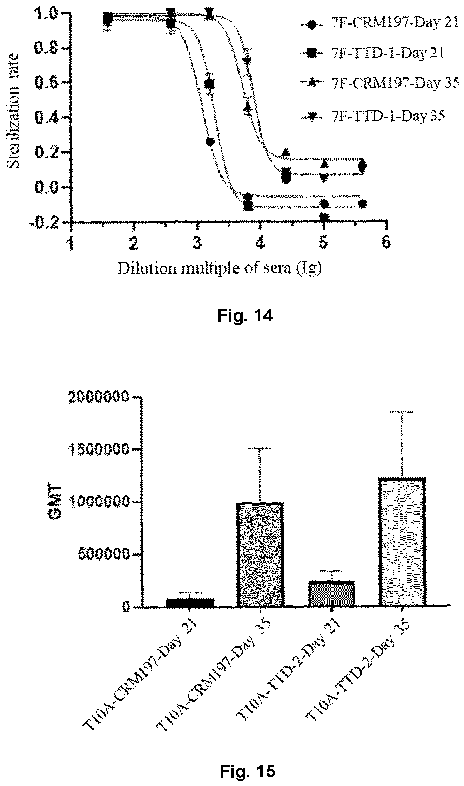

Immune sera of two groups of T7F-CRM197 and T7F-TTD-1 on Day 21 and Day 35 are subjected to OPA test. The results are shown in Figure 14, the IC₅₀ of the immune sera of T7F-CRM197 and T7F-TTD-1 on Day 21 are 1213 and 1925, respectively; the IC₅₀ of the immune sera of T7F-CRM197 and T7F-TTD-1 on Day 35 are 5362 and 7975, respectively. The results indicate that the specific antibodies against the serotype 7F polysaccharides induced by the TTD-1 carrier protein conjugate are not lower than the CRM197 carrier protein conjugate.

### Example 11: immunoenhancing effect of carrier protein TTD on the pneumococcal serotype 10A polysaccharide

Provided are comparisons of the Streptococcus pneumoniae serotype 10A (T 10A) polysaccharide-TTD-2 conjugates with polysaccharide-CRM197 conjugates. Mice were immunized with the polysaccharide-CRM197 conjugates and the polysaccharide-TTD-2 conjugates, and the antibody titers were evaluated on Day 21 and Day 35, respectively, and the results are shown in Figure 15. On Day 35, the serotype-10A-polysaccharide-TTD-2 conjugate can induce higher antibody titers than the polysaccharide-CRM197 conjugates.

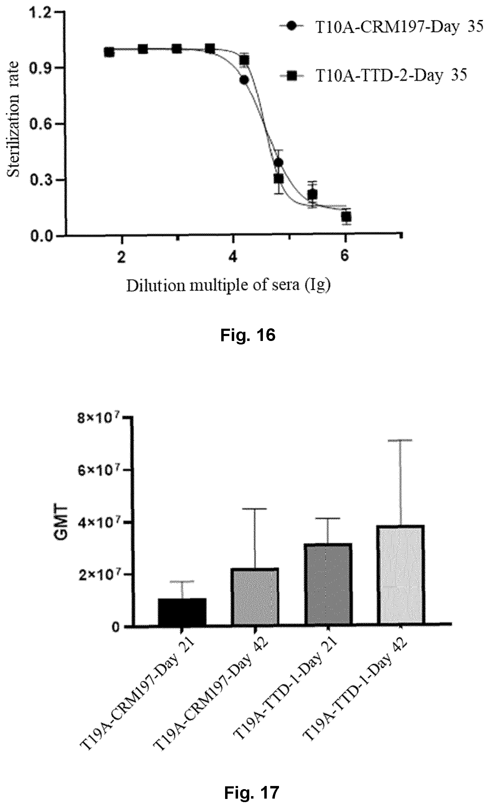

The immune sera of the two groups of T10A-CRM197 and T10A-TTD-2 on Day 35 were subjected to OPA test. The results are shown in Figure 16, the IC₅₀ of the immune sera of 10A-CRM197 and 10A-TTD-2 on Day 35 are 23264 and 30709, respectively. The results indicate that the TTD-2 carrier protein conjugate induces equivalent specific antibodies against the serotype 10A polysaccharides to the CRM197 carrier protein conjugate.

### Example 12: immunoenhancing effect of carrier protein TTD on the pneumococcal serotype 19A polysaccharide

By comparing the immune effect of the Streptococcus pneumoniae serotype 19A (T19A) polysaccharide-TTD conjugate with the Streptococcus pneumoniae serotype 19A polysaccharide-CRM197, the immune enhancement effect of this serotype of Streptococcus pneumoniae polysaccharide-TTD in New Zealand white rabbits was analyzed.

The New Zealand white rabbits were immunized with the serotype-19A-polysaccharide-CRM197 conjugate and the serotype-19A-polysaccharide-TTD-1 conjugate, and the antibody titers on Day 21 and Day 42 were evaluated respectively. The results are shown in Figure 17, on Day 42, the polysaccharide-TTD conjugate can induce higher antibody titers.

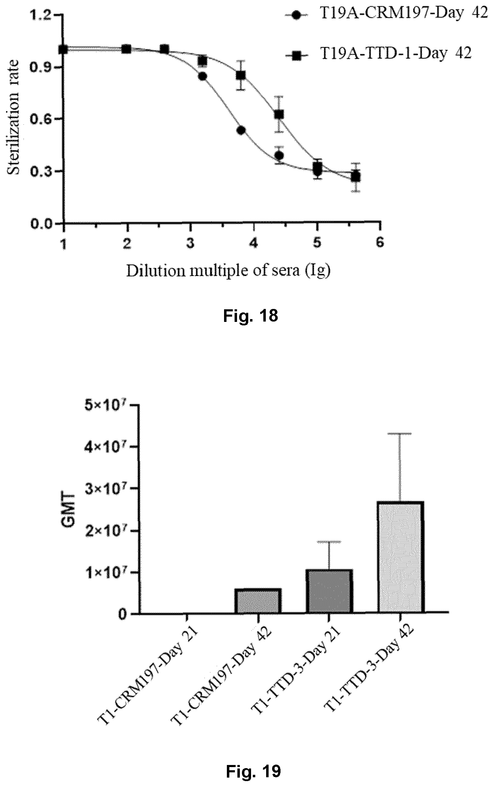

The immune sera of the two groups of T19A-CRM197 and T19A-TTD-1 groups on Day 42 were subjected to OPA test. The results are shown in Figure 18, the IC₅₀ of the immune sera of 19A-CRM197 and 19A-TTD-1 on Day 42 are 8617 and 35780, respectively. The results indicate that TTD-1 carrier protein conjugates induce significantly more specific antibodies against the serotype 19A polysaccharides than CRM197 carrier protein conjugates.

### Example 13: immunoenhancing effect of carrier protein TTD on the pneumococcal serotype 1 polysaccharide

Provided are comparisons of the serotype 1 (T1) of Streptococcus pneumoniae polysaccharide-TTD-3 conjugates with polysaccharide-CRM197 conjugates. Large rabbits were immunized with polysaccharide-CRM197 conjugates and polysaccharide-TTD-3 conjugates, and the antibody titers were evaluated on Day 21 and Day 42, respectively. The results are shown in Figure 19, on Day 42, the polysaccharide-TTD conjugate can induce higher antibody titers.

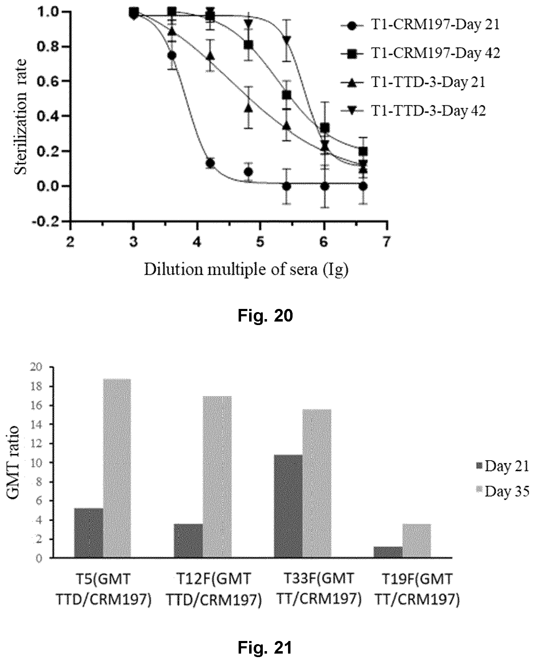

Immune sera of the two groups of T1-CRM197 and T1-TTD-3 on Day 21 and Day 42 were subjected to OPA test. The results are shown in Figure 20, the IC₅₀ of the immune sera of T1-CRM197 and T1-TTD-3 on Day 21 are 6523 and 35571, respectively; the IC₅₀ of the immune sera of T1-CRM197 and T1-TTD-3 on Day 42 are 193732 and 493114, respectively. The results indicate that TTD-3 carrier protein conjugates induce significantly more specific antibodies against the serotype 1 polysaccharides than CRM197 carrier protein conjugates.

### Example 14: immunoenhancing enhancement effect of carrier protein TTD on the pneumonia polysaccharides of other serotype

For other serotypes of Streptococcus pneumoniae polysaccharide conjugates, such as the serotypes 5, 12F, 33F, 19F, mice were immunized with polysaccharide-CRM197 conjugates and polysaccharide-TTD conjugates, and the antibody titers were evaluated on Day 21 and Day 35, respectively (as shown in Figure 21). On Day 35, the polysaccharide-TTD conjugate induce higher antibody titers.

The present invention also compares the immune enhancement effect of TTD protein on polysaccharides of other serotypes of pneumonia, and finds that TTD protein can induce higher immunogenicity than CRM197. For example, mice were immunized with polysaccharide-CRM197 conjugates and polysaccharide-TTD conjugates, and the antibody titers were evaluated on Day 21 and Day 35, respectively, and on Day 35, the ratio of the antibody titer induced by the polysaccharide-TTD conjugate to that of the polysaccharide-CRM197 conjugate can reach 2-20 times, which further illustrates the advantage of TTD as a carrier protein.

### Example 15: Immunogenicity of polysaccharide-TTD variant conjugates

Design of TTD variants: based on protein sequences 01, 02, and 03, amino acids of different lengths at the N-terminal were truncated, and the C-terminal sequence was retained.

Immunogenicity of polysaccharide-TTD variant conjugates: the mice were immunized with the serotype X polysaccharide-CRM197 conjugate (TX-CRM197 group) and the serotype X polysaccharide-TTD conjugate (X-TTD), and the antibody titers on Day 21 and Day 35 were evaluated respectively. On Day 35, the polysaccharide-TTD conjugate induce more antibody titers. X is any one serotype of Streptococcus pneumoniae polysaccharides.

The immune sera of the two groups of TX-CRM197 and TX-TTD on Day 35 were subjected to OPA test. The results indicate that TTD carrier protein conjugates induce significantly more specific antibodies against the serotype X polysaccharides than CRM197 carrier protein conjugates.

The foregoing detailed description has been offered by way of explanation and example, not to limit the scope of the appended claims. Variations on the presently recited embodiments of this application will be apparent to those of ordinary skill in the art and remain within the scope of the appended claims and their equivalents.

## Claims

1. A tetanus toxin protein variant, comprising the segment C of the tetanus toxin protein and the partial segment of the translocation region of the tetanus toxin protein.

2. The tetanus toxin protein variant according to claim 1, wherein the partial segment of the translocation region comprises the T cell epitope P2 of the translocation region of the tetanus toxin protein.

3. The tetanus toxin protein variant according to any one of claims 1-2, wherein the partial segment of the translocation region comprises amino acids 829-864 of the tetanus toxin protein.

4. The tetanus toxin protein variant according to any one of claims 1-3, wherein the partial segment of the translocation region comprises the amino acid sequence shown in any one of SEQ ID NO: 7-8.

5. The tetanus toxin protein variant according to any one of claims 1-4, wherein the segment C of the tetanus toxin protein comprises the amino acid sequence shown in SEQ ID NO:3.

6. The tetanus toxin protein variant according to any one of claims 1-5, comprising the amino acid sequence shown in any one of SEQ ID NO: 1-2.

7. A polysaccharide protein conjugate comprising a polysaccharide from Streptococcus pneumoniae and a truncated tetanus toxin protein (TTD).

8. The polysaccharide protein conjugate according to claim 7, wherein the polysaccharide is derived from Streptococcus pneumoniae capsular polysaccharide.

9. The polysaccharide protein conjugate according to any one of claims 7-8, wherein the polysaccharide has more than one serotype of Streptococcus pneumoniae.

10. The polysaccharide protein conjugate according to any one of claims 7-9, wherein the polysaccharide is selected from any serotype of the group of Streptococcus pneumoniae serotype consisting of: 1, 2, 3, 4, 5, 6A, 6B, 7F, 8, 9N, 9V, 10A, 11A, 12F, 14, 15B, 17F, 18C, 19A, 19F, 20, 22F, 23F, and 33F.

11. The polysaccharide protein conjugate according to any one of claims 7-10, wherein the truncated tetanus toxin protein comprises a segment C of the tetanus toxin protein.

12. The polysaccharide protein conjugate according to any one of claims 7-11, wherein the truncated tetanus toxin protein comprises the segment C of the tetanus toxin protein, wherein the polysaccharide selected from any serotype of the group of Streptococcus pneumoniae serotype consisting of: 1, 3, 5, 6A, 6B, 7F, 10A, 12F, 15B, 19A, 19F, and 33F.

13. The polysaccharide protein conjugate according to any one of claims 7-12, wherein the segment C of the tetanus toxin protein comprises the amino acid sequence shown in SEQ ID NO:3.

14. The polysaccharide protein conjugate according to any one of claims 7-13, wherein the truncated tetanus toxin protein comprises the segment C of the tetanus toxin protein and a partial segment of the translocation region of the tetanus toxin protein.

15. The polysaccharide protein conjugate according to claim 14, wherein the partial segment of the translocation region comprises the universal T cell epitope P2 of the translocation region of the tetanus toxin protein.

16. The polysaccharide protein conjugate according to any one of claims 14-15, wherein the partial segment of the translocation region comprises amino acids 829-864 of the tetanus toxin protein.

17. The polysaccharide protein conjugate according to any one of claims 14-16, wherein the partial segment of the translocation region comprises the amino acid sequence shown in any one of SEQ ID NO: 7-8.

18. The polysaccharide protein conjugate according to any one of claims 7-17, wherein the truncated tetanus toxin protein comprises the amino acid sequence shown in SEQ ID NO:1-3.

19. The polysaccharide protein conjugate according to any one of claims 7-18, wherein the mass ratio of the polysaccharide to the truncated tetanus toxin protein is 0.4-2.5.

20. The polysaccharide protein conjugate according to any one of claims 10-19, wherein the mass ratio of the Streptococcus pneumoniae serotype 1 polysaccharide to the truncated protein is 0.4-2.5.

21. The polysaccharide protein conjugate according to any one of claims 10-20, wherein the mass ratio of the Streptococcus pneumoniae serotype 3 polysaccharide to the truncated protein is 0.4-2.5.

22. The polysaccharide protein conjugate according to any one of claims 10-21, wherein the mass ratio of the Streptococcus pneumoniae serotype 5 polysaccharide to the truncated protein is 0.4-2.5.

23. The polysaccharide protein conjugate according to any one of claims 10-22, wherein the mass ratio of the Streptococcus pneumoniae serotype 6A polysaccharide to the truncated protein is 0.4-2.5.

24. The polysaccharide protein conjugate according to any one of claims 10-23, wherein the mass ratio of the Streptococcus pneumoniae serotype 6B polysaccharide to the truncated protein is 0.4-2.5.

25. The polysaccharide protein conjugate according to any one of claims 10-24, wherein the mass ratio of the Streptococcus pneumoniae serotype 7F polysaccharide to the truncated protein is 0.4-2.5.

26. The polysaccharide protein conjugate according to any one of claims 10-25, wherein the mass ratio of the Streptococcus pneumoniae serotype 10A polysaccharide to the truncated protein is 0.4-2.5.

27. The polysaccharide protein conjugate according to any one of claims 10-26, wherein the mass ratio of the Streptococcus pneumoniae serotype 12F polysaccharide to the truncated protein is 0.4-2.5.

28. The polysaccharide protein conjugate according to any one of claims 10-27, wherein the mass ratio of the Streptococcus pneumoniae serotype 15B polysaccharide to the truncated protein is 0.4-2.5.

29. The polysaccharide protein conjugate according to any one of claims 10-28, wherein the mass ratio of the Streptococcus pneumoniae serotype 19A polysaccharide to the truncated protein is 0.4-2.5.

30. The polysaccharide protein conjugate according to any one of claims 10-29, wherein the mass ratio of the Streptococcus pneumoniae serotype 19F polysaccharide to the truncated protein is 0.4-2.5.

31. The polysaccharide protein conjugate according to any one of claims 10-30, wherein the mass ratio of the Streptococcus pneumoniae serotype 33F polysaccharide to the truncated protein is 0.4-2.5.

32. The polysaccharide protein conjugate according to any one of claims 7-31, wherein the polysaccharide and the protein variant can be conjugated by a conjugation method.

33. The polysaccharide protein conjugate according to claim 32, wherein the conjugation method comprises any one of the following methods: hydrogen bromide method, CDAP method, reducing amine method.

34. A method for improving immunogenicity, comprising the step of: providing a polysaccharide protein conjugate comprising the polysaccharide from Streptococcus pneumoniae and the truncated tetanus toxin protein.

35. The method according to claim 34, wherein the polysaccharide is derived from Streptococcus pneumoniae capsular polysaccharide.

36. The method according to any one of claims 34-35, wherein the polysaccharide has more than one serotype of Streptococcus pneumoniae.

37. The polysaccharide protein conjugate according to any one of claims 34-36, wherein the polysaccharide is selected from any serotype of the group of Streptococcus pneumoniae serotypes consisting of: 1, 2, 3, 4, 5, 6A, 6B, 7F, 8, 9N, 9V, 10A, 11A, 12F, 14, 15B, 17F, 18C, 19A, 19F, 20, 22F, 23F, and 33F.

38. The polysaccharide protein conjugate according to any one of claims 34-37, wherein the truncated tetanus toxin protein comprises a segment C of the tetanus toxin protein.

39. The method according to claims 34-38, wherein the truncated tetanus toxin protein comprises the segment C of the tetanus toxin protein, wherein the polysaccharide is any serotype of Streptococcus pneumoniae selected from the group consisting of: 1, 3, 5, 6A, 6B, 7F, 10A, 12F, 15B, 19A, 19F, and 33F.

40. The method according to any one of claims 38-39, wherein the segment C of the tetanus toxin protein comprises the amino acid sequence shown in SEQ ID NO:3.

41. The method according to any one of claims 34-40, wherein the truncated tetanus toxin protein comprises a segment C of the tetanus toxin protein and a partial segment of the translocation region of the tetanus toxin protein.

42. The method according to claim 41, wherein the partial segment of the translocation region comprises the universal T cell epitope P2 of the translocation region of the tetanus toxin protein.

43. The method according to any one of claims 41-42, wherein the partial segment of the translocation region comprises amino acids 829-864 of the tetanus toxin protein.

44. The method according to any one of claims 34-43, wherein the truncated tetanus toxin protein comprises the amino acid sequence shown in SEQ ID NO: 1-3.

45. The method according to any one of claims 34-44, wherein the mass ratio of the Streptococcus pneumoniae polysaccharide to the truncated tetanus toxin protein is 0.4-2.5.

46. The method according to any one of claims 37-45, wherein the mass ratio of the Streptococcus pneumoniae serotype 1 polysaccharide to the truncated protein is 0.4-2.5.

47. The method according to any one of claims 37-46, wherein the mass ratio of the Streptococcus pneumoniae serotype 3 polysaccharide to the truncated protein is 0.4-2.5.

48. The method according to any one of claims 37-47, wherein the mass ratio of the Streptococcus pneumoniae serotype 5 polysaccharide to the truncated protein is 0.4-2.5.

49. The method according to any one of claims 37-48, wherein the mass ratio of the Streptococcus pneumoniae serotype 6A polysaccharide to the truncated protein is 0.4-2.5.

50. The method according to any one of claims 37-49, wherein the mass ratio of the Streptococcus pneumoniae serotype 6B polysaccharide to the truncated protein is 0.4-2.5.

51. The method according to any one of claims 37-50, wherein the mass ratio of the Streptococcus pneumoniae serotype 7F polysaccharide to the truncated protein is 0.4-2.5.

52. The method according to any one of claims 37-51, wherein the mass ratio of the Streptococcus pneumoniae serotype 10A polysaccharide to the truncated protein is 0.4-2.5.

53. The method according to any one of claims 37-52, wherein the mass ratio of the Streptococcus pneumoniae serotype 12F polysaccharide to the truncated protein is 0.4-2.5.

54. The method according to any one of claims 37-53, wherein the mass ratio of the Streptococcus pneumoniae serotype 15B polysaccharide to the truncated protein is 0.4-2.5.

55. The method according to any one of claims 37-54, wherein the mass ratio of the Streptococcus pneumoniae serotype 19A polysaccharide to the truncated protein is 0.4-2.5.

56. The method according to any one of claims 37-55, wherein the mass ratio of the Streptococcus pneumoniae serotype 19F polysaccharide to the truncated protein is 0.4-2.5.

57. The method according to any one of claims 37-56, wherein the mass ratio of the Streptococcus pneumoniae serotype 33F polysaccharide to the truncated protein is 0.4-2.5.

58. The method according to any one of claims 34-57, wherein the method comprises conjugating the polysaccharide and the protein variant by a conjugation method.

59. The method according to claim 58, wherein the conjugation method comprises any one of the following methods: hydrogen bromide method, CDAP method, reductive amination method.

60. The method according to any one of claims 34-59, wherein the improving the immunogenicity of the bacterial polysaccharide comprises that the polysaccharide protein conjugate has a higher immunogenicity than the polysaccharide-CRM197 conjugate.

61. The method according to any one of claims 34-60, wherein the higher immunogenicity is detected after an animal immunization experiment.

62. The method according to claim 61, wherein the animal immunization experiment comprises the following steps: preparing the polysaccharide protein conjugate and adjuvant according to any of claims 7-33 into an immune antigen.

63. The method according to claim 62, wherein the injection method of the immune antigen comprises intraperitoneal injection, subcutaneous injection, intramuscular injection and/or intravenous injection.

64. The method according to any one of claims 61-63, wherein the animal immunization experiment comprises the following steps: detecting the antibodies in the sera of the obtained immunized animals by ELISA.

65. The method according to any one of claims 61-64, wherein the animal immunization experiment comprises the following steps: performing an opsonophagocytic killing assay on the serum of the obtained immunized animals.

66. The method of any one of claims 61-65, wherein the animal comprises a mouse, a rat and/or a rabbit.

67. The method according to any one of claims 62-66, wherein the adjuvant comprises aluminum hydroxide, aluminum phosphate and/or Freund's adjuvant and the like.

68. A nucleic acid molecule, comprising a sequence encoding the tetanus toxin protein variant of any one of claims 1-6.

69. The nucleic acid molecule according to claim 68, comprising the nucleotide sequences shown in SEQ ID NO: 4-6.

70. A vector, comprising the nucleic acid molecule of any one of claims 68-69.

71. A cell, comprising the nucleic acid molecule of any one of claims 68-69 or the vector of claim 70.

72. A carrier protein, comprising the tetanus toxin protein variant according to any one of claims 1-6.

73. A pharmaceutical composition, comprising the polysaccharide protein conjugate of any one of claims 7-33 and optionally a pharmaceutically acceptable adjuvant

74. Use of the tetanus toxin protein variant according to any one of claims 1-6 for the preparation of a medicament.

75. Use of the polysaccharide conjugate according to any one of claims 7-33 for the preparation of a medicament.

76. The use according to any one of claims 74-75, wherein the medicament is used for preventing and/or treating Streptococcus pneumoniae disease.

77. The use according to claim 76, wherein the Streptococcus pneumoniae disease comprises pneumonia, sepsis, meningitis and/or otitis media.

78. A vaccine, comprising the polysaccharide protein conjugate of any one of claims 7-33, the pharmaceutical composition of claim 73 and/or optionally a pharmaceutically acceptable adjuvant.

79. A method for preventing disease caused by Streptococcus pneumoniae infection, comprising administering the polysaccharide protein conjugate of any one of claims 7-33, the nucleic acid molecule of any one of claims 68-69, the vector of claim 70, the cell of claim 71, the carrier protein of claim 72, the pharmaceutical composition of claim 73 and/or the vaccine of claim 78 to a subject in need.

80. The method of claim 79, wherein the Streptococcus pneumoniae disease comprises pneumonia, sepsis, meningitis and/or otitis media.

81. Use of the polysaccharide conjugate according to any one of claims 7-33 as an antigen for the preparation of a antibody.

82. The use according to claim 81, wherein the antibody is used for diagnostic typing of an isolated bacterial strain.

83. A kit, comprising the polysaccharide conjugate of any one of claims 7-33.

84. The kit according to claim 83, wherein the kit is used for the diagnostic typing of an isolated bacterial strain.
